# EUROPEAN PATENT APPLICATION

(11) **EP 4 729 583 A2**
(43) Date of publication of application: **22.04.2026**
(21) Application number: 25216161.7
(22) Date of filing: 19.08.2022
(51) Int. Cl.: C09B 67/44

(54) **CYANINE DYES AND THEIR USAGE FOR IN VIVO STAINING OF MICROORGANISMS AND OTHER LIVING CELLS**

(30) Priority: 20.08.2021 EP 21192451
(62) Divisional of application: 22191279.3
(71) Applicant: Philipps-Universität Marburg, 35037 Marburg (DE)
(72) Inventor: Vazquez, Olalla, 35037 Marburg (DE); Schulte, Leon, 35037 Marburg (DE); Plocher, Benedikt, 61118 Bad Vilbel (DE); Linden, Greta, 80807 München (DE)
(74) Representative: Stumpf, Peter

(57) **Abstract**

The invention provides new cyanine dyes for staining living cells, providing fluorescence emission in red, green and yellow, thus allowing "multichannel" staining. The dyes are binding to nucleic acids and allow the observation of the stained cells, for the staining does not negatively interfere with the viability of the stained cells. The inventive dyes thus can advantageously be used for pathogen-host investigations or any other type of investigation of cell-cell-interactions, for the natural behavior of the stained cells (microorganisms, pathogens etc.) can easily be observed.

## Description

The invention relates to cyanine dyes, the production of cyanine dyes and their use for in vivo-staining of microorganisms, especially pathogens, and other living cells.

### Description

### Field of the invention

Several important microorganisms are still difficult to modify for GFP expression for the purpose of investigating them. This is particularly true for the growing number of multi-drug resistant bacteria, posing an emerging threat to patients in clinics worldwide. Specifically, these strains may acquire resistances to the antibiotics used to select transformants carrying the GFP transgene. Thus, the investigation of host cell interplay with drug-resistant bacteria by means of fluorescent labelling using GFP is not applicable generally. The expression "in vivo"-staining" as is used herein has the meaning of staining living cells without major negative interference on their viability so that their live behaviour, e.g. their interactions with other cells may be observed by methods known to the person skilled in the art after being stained, e.g. via flow cytometry or fluorescence microscopy etc. The surprising effect that the in vivo-staining is not negatively interferring with the viability of the stained cells is experimentally proven by comparative growth studies of stained cells at distinct dye-concentration in comparison with reference compounds, e.g. DMSO (preferred method with respect to microbial cells) and/or by application of resazurin-based assay to the cells several hours after being stained (preferred method for non-microbial cells). These comparative viability studies resp. resazurin-based assays are performed either with the cells one wants to investigate by use of in vivo staining or with HeLa cells or other standard cell lines known to the person skilled in the art (or, for example, *E. Coli* as standard microbial model organism with respect to microbial cells).

### Background of the technology

In vivo staining of cells with cyanine dyes as is defined herein (especially multiple-colour staining of one or more samples of cells), exerting the surprising effect that the staining with cyanine dyes does not negatively influence the viability of the stained cells, is unknown heretofore. Interference of staining with the viability of the stained cells is mostly not addressed at all, as can be exemplarily seen within the documents referenced below.

US 2016/340717 A1 reveals a dye resp. probe for detecting bacterial or viral endonucleases which is composed of an oligonucleotide, a fluorophore connected to the oligonucleotide and a fluorescence quencher, also being connected to the oligonucleotide. Fluorescence is emitted only upon cleavage of the dye/probe. US 2016/340717 A1 nowhere reveals that it is intended to keep cells living after being stained by the dye/probe. So, US 2016/340717 A1 is not addressing the problem to be solved by the invention revealed herein.

Jieqiong Qiu et al reveal combination probes with intercalating anchors and proximal fluorophores for DNA and RNA detection (cf. Nucleic Acis Research, 2016, Vol. 44, No. 17 - doi: 10.1093/narlgkw579*).* This article describes the usage of an anchor-dye and a reporter dye, i. e. two dyes intercalating into DNA/RNA for achieving the desired staining. Nowhere is revealed that the dyes applied do not negatively interfere with the viability of the stained cells. The technical teaching of this document is completely different from the invention revealed herein below.

US 2011/0262904 A1 describes dyes having a good solubility in water and a good live cell permeability. But nowhere within US 2011/0262904 A1 it is stated or claimed that the dyes do not negatively interfere with the viability of the stained cells. Staining cells without negatively affecting their viability is not addressed at all in US 2011/0262904 A1, indicating that this problem is not concerned about. In US 2011/0262904 A1 it is only important that the dyes can permeate the membranes of live cells for staining, but the destiny of the cells, their viability upon being stained is of no importance. The technical teaching of this document is also completely different from the invention revealed herein below.

Similarly, WO 2013/189043 A1 and US 2015/0185182 A1 only describe live cells being stained, but the effect of the staining upon viability of the stained cells is not addressed at all. The technical teaching of this document is also completely different from the invention revealed herein below.

US 2010/0151509 A1 is revealing staining and counting leucocytes, for which purpose it is not necessary that stained cells stay alive after having been stained. Consequently interference of staining with the viability of the stained cells (leucocytes) is not addressed at all in US 2010/0151509 A1. Leucocytes by nature cannot multiply (grow) like other cells or microorganisms, so that this document is dealing with a totally different subject for which the parameter "viability" is not applicable at all. Thus, the technical teaching of this document is also completely different from the invention revealed herein below.

Tinghan Zhao et al. describe a very special dye being composed of a tetramethylrhodamin moiety being chemically bound to a specific MPP (mitochondria penetrating protein), rFrFrF.

Rhodamine dyes are known mitochondria markers by themselves, e.g. tetramethylrhodamine ethyl ester (TMRE), which is commercially available for the purpose of staining mitochondria in living cells. It is structural very close to TAMRA. The known applicability of TMRE for staining of living cells is misleading to the application of dyes of similar structure - like TAMRA. Also, TMRE is not interacting with the DNA and it is not a light-up dye, so background is always higher than with cyanine dyes. Thus, application of cyanine dyes for staining according to the invention will improve the background-to-signal ratio.

The article (cf. Bioconjugate Chem. 2019, 30, 2312 - 2316) provides only one specific dye-component (TAMRA), belonging to a class of dyes which is known to be usable for marking/staining mitochondria, and no hint at all that conjugates for staining mitochondria may be possible to be composed of cyanine dyes and MPP's. Cyanine dyes were unknown heretofore to be applicable for marking/staining mitochondria in live cells without negatively interfering with the viability of the stained cells.

Patent application EP 3 572 468 A1 reveals cyanine dyes binding to DNA and exerting fluorescence upon excitation with light of suitable wavelength, e.g. cyanine dye **3,** exerting red fluorescence. Heretofore these compounds were only applied solely.

Now, it is found and revealed herein that it is possible to combine multiple DNA binding cyanine dyes exerting different fluorescence colours within one experiment without negatively interfering with the viability of the stained cells, thus providing the possibility of multichannel observation (red, green and yellow fluorescence) of interactions of different cells with each other. Also, new cyanine dyes of different structure, also binding to DNA, have been found, synthesized and investigated, which are also exerting red, yellow resp. green fluorescence upon excitation with light of suitable wavelength, e.g. cyanine dyes **1,** exerting green fluorescence, **2,** exerting yellow fluorescence, **15,** exerting green fluorescence, **16,** exerting yellow fluorescence, **17,** exerting red fluorescence, **18,** exerting green fluorescence, **19,** exerting yellow fluorescence and **20,** exerting red fluorescence.

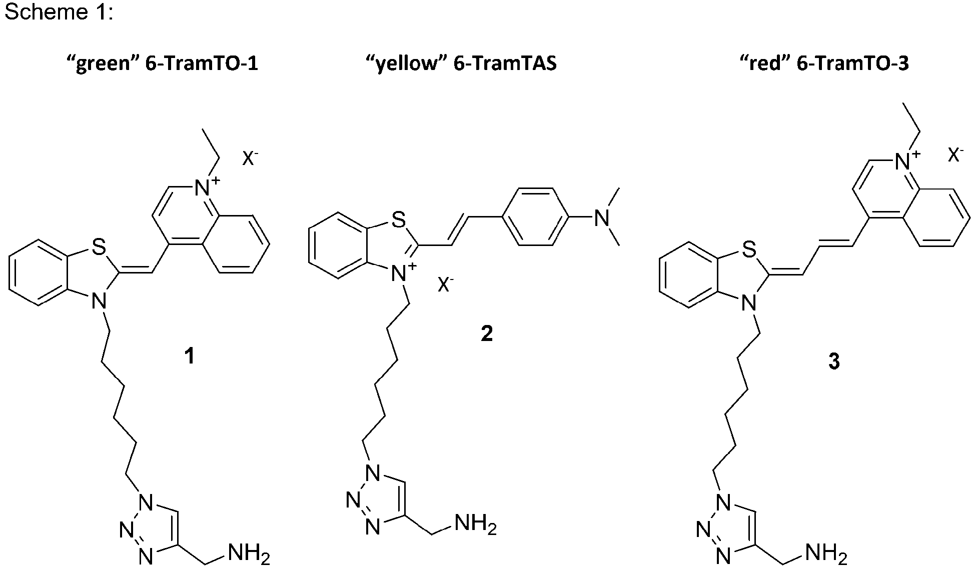

### Content of the invention

An objective of the present invention is to provide a method for staining living cells in such a way that different sample populations of cells are stained with different (at least two) cyanine dyes (most preferred cyanine dyes which are binding to nucleic acid) which are not reducing the viability of the stained cells, so that interaction of the stained population of cells can be observed via multichannel fluorescence microscopy.

A second objective of the present invention is to provide substances (cyanine dyes) that can be used for in vivo-staining of cells, especially microorganisms, without significantly reducing the viability of the stained cells (e. g. microbes, yeasts) according to the inventive method for staining living cells. The term "microorganism" (and all of its grammatical forms) as used with regard to the present invention comprehends not only all types of bacteria, fungi etc., but also pathogenic structures of all types. The terms "microorganism", "pathogen", "bacteria" etc. are therefore synonymously used throughout this whole revelation of the current invention. The term "cell" (and all of its grammatical forms) as used with regard to the present invention comprehends not only cells of microorganisms and its synonyms but also cells of vertebrates and plants.

In order to achieve the first objection a method for staining living cells is revealed herein applying at least two nucleic acid binding cyanine dyes with different fluorescence emission wavelengths which are not reducing the viability of the stained cells substantially.

A cyanine dye (most preferredly a cyanine dye which is binding to nucleic acid) is not reducing the viability of cells substantially according to the invention if: a) microbial (e. g. E. Coli) growth after 10h under identical breeding conditions at dye concentration of 2.5 µM is at least 90% compared to DMSO-reference and at dye concentration of 5 µM is at least 70% compared to DMSO-reference and/or b) growth of HeLa cells under identical breeding conditions at dye concentration of 10 µM is at least 50% of cell viability compared to reference (for example: concentration of 10 µM TriTO-1 leads to viability of about 62%, concentration of 10 µM 6-TramTO-1 (**1**) leads to viability of about 97%, concentration of 10 µM 6-TramTO-3 (**3**) leads to viability of about 97%).

It is well known to the person of ordinary skill in the art how to perform experimental measurements of microbial growth or cell viability as are described in the proceeding paragraph, for example regarding choosing the suitable microorganism or cell type, breeding conditions (growth medium, breeding temperature, surrounding atmosphere etc.), choice and applied amount of reference compounds if necessary (e. g. DMSO, MeCN), defining further reference conditions etc.

In order to achieve the second objective, the present invention provides new types of cyanine dyes of formula (**I**), 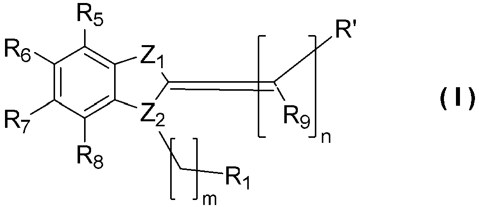 allowing in vivo staining of pathogens without negatively interfering with the viability of the pathogens.

The above objectives are achieved through the independent claims 1, 4 and 9 through 12. The dependent claims reveal additional advantageous embodiments of the invention.

The cyanine dyes from previous research exhibited mainly red fluorescence, enabling them to be detected in the far-red fluorescence channels of fluorescence microscopes or other fluorescence detection apparatuses only. The work revealed herein has led to new compounds (e. g. cyanine dyes **1, 2**) exerting excellent characteristics for live-staining (in vivo-staining) of cells, i.e. the new compounds exert distinctively less interference with the viability of cells upon staining in that they can also be used for staining cells of vertebrates, e.g. HeLa cells. The new compounds exert red, green and yellow fluorescence, thus providing the possibility of staining live cells with several cyanine dyes exerting different fluorescence colours at the same time and thus allowing a multichannel fluorescence observation of the behaviour of, for example, living microbial cells, to a much greater extent than previously known. Alternatively different species of microbial cells may be stained with different cyanine dyes, exerting different fluorescence, and being simultaneously observed within the same sample at the same time. This process of staining cells, basically without reducing the viability of the cells, is herein referred to as "in vivo staining".

Scheme 2 shows cyanine dyes according to the state-of-the-art exerting green, yellow and red fluorescence upon excitation with light of suitable wavelength, which are used as control samples within the experiments. Scheme 3 shows further examples of cyanine dyes according to the state of the art, being modified regarding substituent **X,** whereat general formula **Ia** represents examples of red fluorescent cyanine dyes and general formula **IIa** represents examples of green fluorescent cyanine dyes.

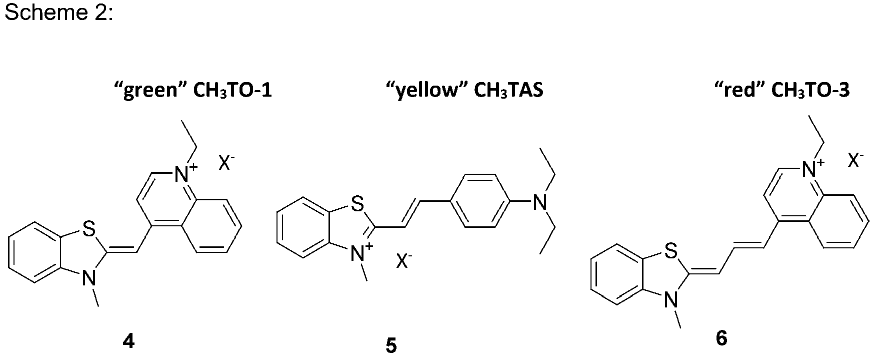

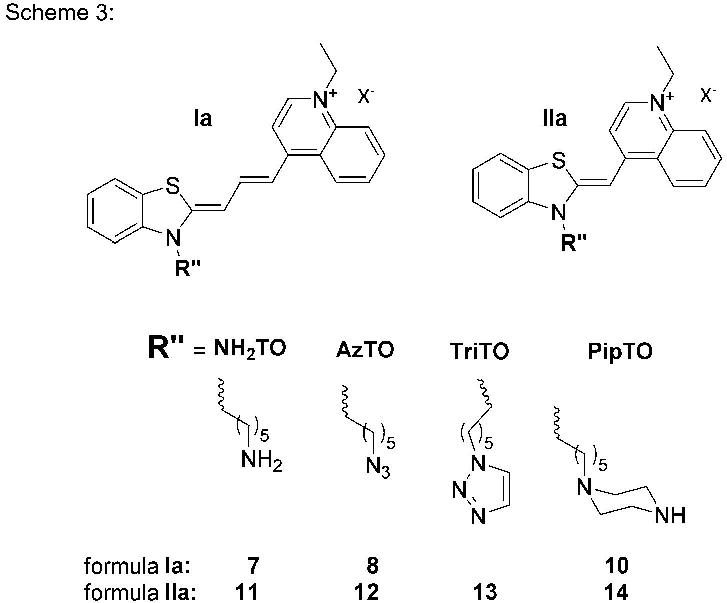

The scope of the invention is comprising compounds where substituent **R"** is (for example): 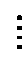 -(CH₂)₅-PPh₃ (e. g. **15** (green fluorescence), **16** (yellow fluorescence), **17** (red fluorescence)). The substituent -PPh₃ at the end of the side chain attached to the N-atom of the thiazole ring is introducing the possibility of staining organelles, especially e.g. mitochondria. This effect was heretofore unknown to be working in combination with cyanine dyes, thus this development, revealed herein, provides the possibility of staining organelles with cyanine dyes.

Following table 1 shows some of the molecular extinction coefficients that are exemplarily measured.

**Table 1: Molar extinction coefficients**

| **Compound** | **solvent** | **Molar extinction coefficient** |
|---|---|---|
| 6-TramTO-3 (**3**) | H₂O | 59403 M⁻¹cm⁻¹ |
| CH₃TO-3 (**6**) | DMSO | 37153 M⁻¹cm⁻¹ |
| 6-TramTAS (**2**) | MeCN | 70463 M⁻¹cm⁻¹ |
| CH₃TAS (**5**) | MeCN | lit. 82200 M⁻¹cm⁻¹ |
| 6-TramTO-1 (**1**) | H₂O | 61813 M⁻¹cm⁻¹ |
| CH₃TO-1 (**4**) | DMSO | lit. 63000 M⁻¹cm⁻¹ |

Therefore, the subject matter of the invention may be summarised as follows.

The subject matter of the invention is a method for staining living cells characterized in that the method comprises the following steps:
i) at least one cyanine dye is applied to at least one sample of cells by adding a solution of the at least one cyanine dye to the at least one sample of cells, whereat
   - the at least one cyanine dye is not reducing the viability of the stained cells substantially, whereat the non-reduction of the viability of the living cells through the at least one cyanine dye is defined by
      - comparable microbial growth measurement against DMSO under identical breeding conditions whereat the cell-count in the sample with the cyanine dye after 10 h breeding under identical conditions at dye concentration of 2.5 µM is at least 94% compared to DMSO-reference and/or at dye concentration of 5 µM is at least 80% compared to DMSO-reference and/or
      - comparable viability measurements of eukariotic cells, for example HeLa cells, under identical breeding
         conditions at dye concentration of 5 µM is at least 85% compared to reference, whereat the reference is either viability of the eukariotic cells, for example HeLa cells, in
         presence of acetonitrile or viability of the pure cells (eukariotic cells, for example HeLa cells) or viability
         of the cells (eukariotic cells, for example HeLa cells), grown in water, thus providing at least one sample of cells, being stained with at least one cyanine dye;
ii) incubating the at least one sample of cells according to step i) for at least 15 minutes, so that stained living cells are existent.

The cells to be stained for investigation (= "sample of cells") and those for determining the non-interference of viability (= "eukariotic cells, for example HeLa cells") may be of the same type or of different type.

The subject matter of the invention also comprises a method for staining living cells as described before, characterized in that the method comprises the following steps i) and ii) and the additional step iii):
i) at least two cyanine dyes are applied to at least two separate
   samples of cells whereat each sample is stained with one of the at least two cyanine dyes by adding a solution of the cyanine dye to the sample of cells, whereat
      - each cyanine dye exerts a fluorescence emission wavelength different from the fluorescence emission wavelengths of all other cyanine dyes being applied and
      - each cyanine dye is not reducing the viability of the stained cells substantially, whereat the non-reduction of the viability of the living cells through the cyanine dye is defined for each cyanine dye by
         - comparable microbial growth measurement against DMSO under identical breeding conditions whereat the cell-count in the sample with the cyanine dye after 10 h breeding under identical conditions at dye concentration of 2.5 µM is at least 94% compared to DMSO-reference and/or at dye concentration of 5 µM is at least 80% compared to DMSO-reference and/or
         - comparable viability measurements of eukariotic cells, for example HeLa cells, under identical breeding
            conditions at dye concentration of 5 µM is at least 85% compared to reference, whereat the reference is either viability of the eukariotic cells, for example HeLa cells, in
            presence of acetonitrile or viability of the pure cells (eukariotic cells, for example HeLa cells) or viability of the cells (eukariotic cells, for example HeLa cells), grown in water,
   thus providing at least two separate samples of cells, each one being stained with at least one cyanine dye;
ii) each sample of cells according to modified step i) is incubated for at least 15 minutes, so that stained living cells are existent;
iii) the at least two separate samples of cells provided according to step ii), each one being stained with at least one cyanine dye, are combined within one sample vessel with each other thus forming one combined sample.

The cells to be stained for investigation (= "samples of cells") and those for determining the non-interference of viability (= "eukariotic cells, for example HeLa cells") may be of the same type or of different type.

The subject matter of the invention also comprises any one method as described before, characterized in that the cyanine dye is a cyanine dye for use in binding to nucleic acid.

The subject matter of the invention also comprises any one method as described before, characterized in that the method comprises the following additional preparation step before step i):
a) obtaining and isolation of at least one sample of cells to be introduced into step i), whereat this obtaining and isolation-step may include one or more steps for concentrating and/or breeding of the cells.

The subject matter of the invention also comprises a cyanine dye for usage in any one method as is described before, the chemical structure of the cyanine dye being according to general formula (**I**), 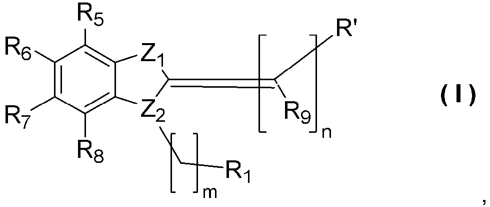 wherein
- R' is a moiety according to formula (**II**) or a moiety according to formula (**III**), 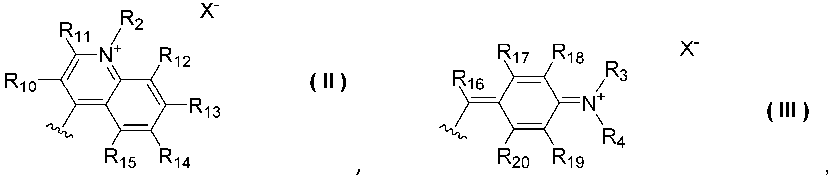
- R₂ is selected from the list comprising H, methyl, ethyl, propyl, iso-propyl, butyl, iso-butyl, tert-butyl, alkyl, alkinyl, alkylidene insofar as substituent R₂ is present according to the choice of R',
- R₃ and R₄ are independently from each other selected from the list comprising H, methyl, ethyl, propyl, iso-propyl, butyl, iso-butyl, tert-butyl, alkyl, alkinyl, alkylidene insofar as substituents R₃, R₄ are present according to the choice of R',
- R₅ to R₂₀ are independently from each other selected from the list comprising the substituents H, halogen, methyl. ethyl, propyl, isopropyl, methoxy, ethoxy, propoxy, amino, methylamino, ethylamino, monoalkylamino, dialkylamino, a nitro group, a sulfo group insofar as any one of substituents R₅ to R₂₀ is present according to the choice of R',
- Z₁ and Z₂ are each independently O, S, or N,
- X⁻ is selected from the list comprising halogenate, sulfate, triflate, trifluoro acetate, difluoro acetate or fluoro acetate,
- n is 1 to 5,
- m is 1 to 10,
   characterized by
- R₁ is independently chosen from the list comprising the substituents triphenylphosphine, substituted triphenylphosphine, 1,2,3-triazol-1-yl, 1,2,3-triazol-2-yl, 1,2,3.triazol-4-(aminomethyl)-1-yl
   mitochondria penetrating peptide - whereat the mitochondria penetrating peptide is chemically bound to the alkyl-chain of length m via peptide-bond or via sulfide-bond or via ester-bond or via thioester-bond -
   if R' is chosen to be a moiety according to formula (**II**);
   or
- R₁ is independently chosen from the list comprising the substituents OH, SH, N₃, NH₂, NH₃⁺, NHR₃, NH₂R₃⁺, NR₂₁R₂₂, NR₂₁R₂₂H⁺ - whereat R₂₁ and R₂₂ are independently from each other chosen from the list comprising methyl, ethyl or propyl -, substituted or non-substituted heterocyclic structure, substituted or non-substituted cyclic structure, triphenylphosphine,
   substituted triphenylphosphine, 1,2,3-triazol-1-yl, 1,2,3-triazol-2-yl,
   substituted 1,2,3-triazol-1-yl, substituted 1,2,3-triazol-2-yl,
   mitochondria penetrating peptide - whereat the mitochondria penetrating peptide is chemically bound to the alkyl-chain of length m via peptide-bond or via sulfide-bond or via ester-bond or via thioester-bond -
   if R' is chosen to be a moiety according to formula (**III**).

The subject matter of the invention also comprises any cyanine dye as is described before in its protonated or deprotonated form, whereat the counterion, resp. counterions, of the protonated or deprotonated form is, resp. are, independently selected from the list comprising halogenate, sulfate, triflate, trifluoro acetate, difluoro acetate or fluoro acetate.

The subject matter of the invention also comprises any cyanine dye as is described before, characterized in that it is for use in binding to oligomeric DNA or primer-DNA or DNA-aptameres.

The subject matter of the invention also comprises a kit for carrying out any one of the methods described above, the kit comprising the cyanine dye and optionally supportive substances.

The subject matter of the invention also comprises the synthesizing method of a cyanine dye as is described above, characterized in that the synthesizing method comprises the following steps:
a) performing a substitution reaction on a precursor compound according to formula (**IV**), 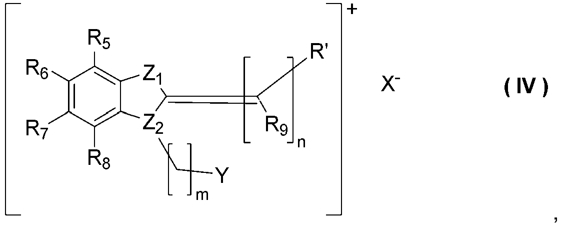 whereat
   the substituent Y in formula (**IV**) is selected from the list comprising the substituents chlorine, bromine, iodine, mesylate, tosylate, fluorinated mesylate, fluorinated tosylate and all other substituents in formula (**IV**) have the same meaning and are chosen in the same way as is denoted in claim 5 regarding formula (**I**) and indices m and n have the same values as are denoted in claim 5 regarding formula (**I**),
   by reacting the precursor compound according to formula (**IV**) in a reaction mixture with a nucleophilic compound, leading to the
   substitution of substituent Y by the nucleophilic compound;

b) extracting the reaction product from the reaction mixture from step a) and
c) purifying the reaction product retrieved from step b).

The subject matter of the invention also comprises a method as described before, characterized in that the nucleophilic compound in step a) is triphenylphosphine or 1,2,3-triazole or a mitochondria penetrating peptide.

The subject matter of the invention also comprises a method as described above, characterized in that the nucleophilic compound in step a) is the azide ion and the reaction product from step a) or the extracted reaction product from step b) or the purified reaction product from step c) is further reacted in an additional step d), the additional step d) being inserted between steps a) and b) or between steps b) and c) or being placed after step c).

The subject matter of the invention also comprises a method as described before, characterized in that the additional step d) comprises the reaction of a precursor compound according to formula (**V**), 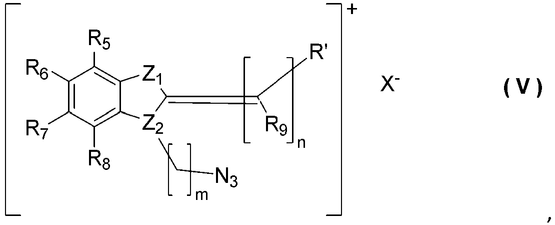
whereat in this case R' is restricted to a moiety according to formula (III),
with substituted or unsubstituted Tris((1-benzyl-4-triazolyl)methyl)amine, "TBTA",
thus forming a cyanine dye according to any one of the preceeding claims 5 through 9 having a substructure where R' is a moiety according to formula (**III**) and R₁ is a substituent with a substituted or non-substituted heterocyclic structure.

The subject matter of the invention also comprises a method as described above, characterized in that the additional step d) comprises the reaction of a precursor compound according to formula (**V**), 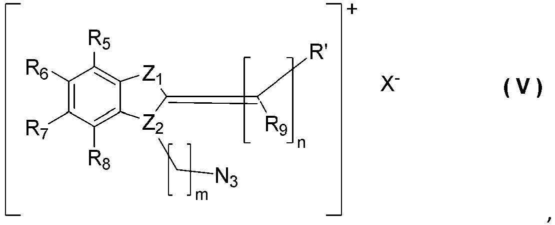
whereat R' is a moiety according to formula (**II**) or according to formula (**III**),
whereat n is 1 or n is 2 or n is 3 or n is 4 or n is 5,
in a copper-catalyzed azide-alkyne cycloaddition reaction with a substituted or unsubstituted alkyne, thus forming a cyanine dye according to any one of the preceeding claims 5 through 9, whereat R₁ is a substituent with a substituted or non-substituted heterocyclic structure.

### Detailed embodiments of the invention

All experimental data revealed herein is provided by way of example only and is not to be understood as being confining the scope of the invention.

### Discussion of toxicity of some exemplary compounds

### Exemplary growth curves of some sample microorganisms, resp. sample cells exemplarily stained

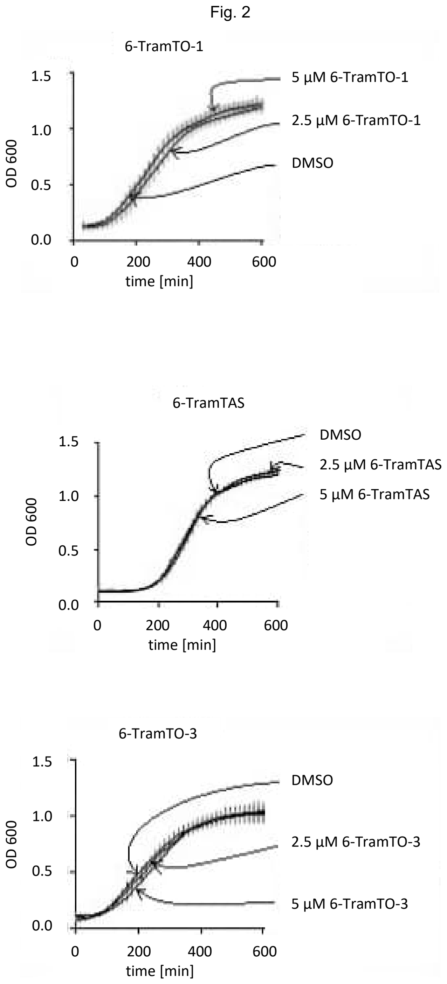

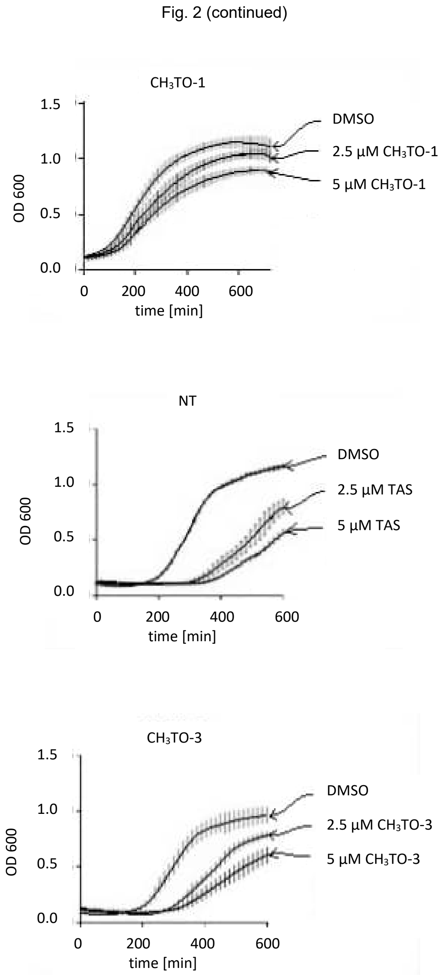

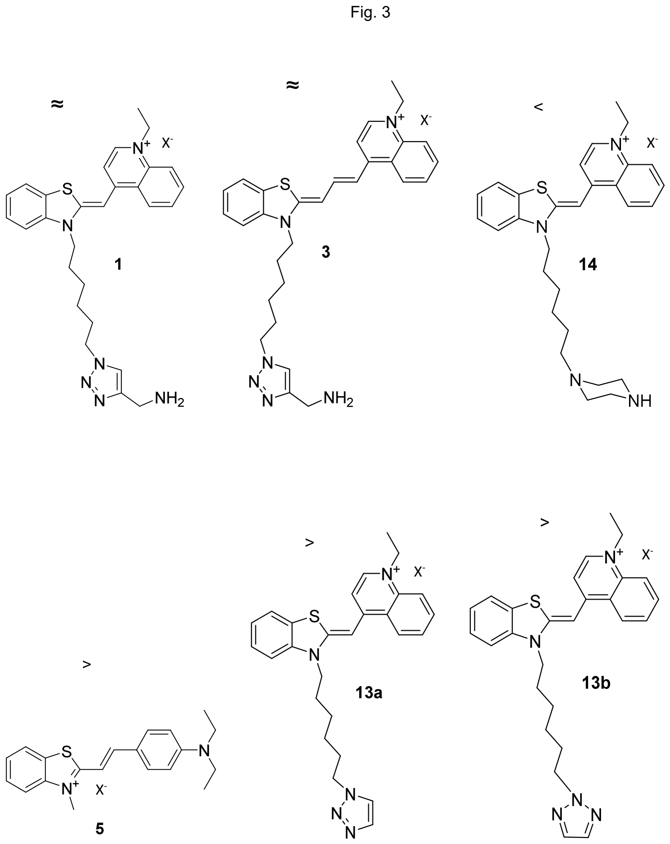

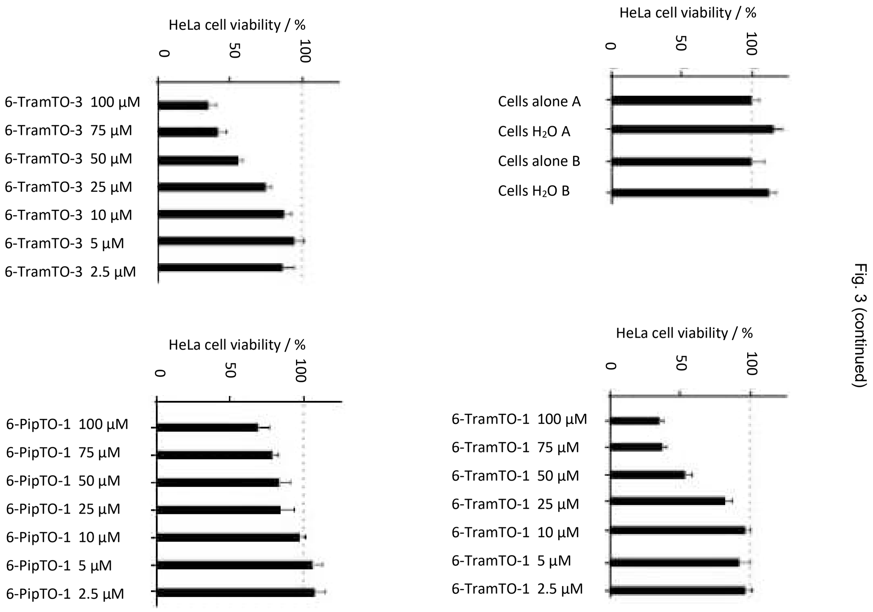

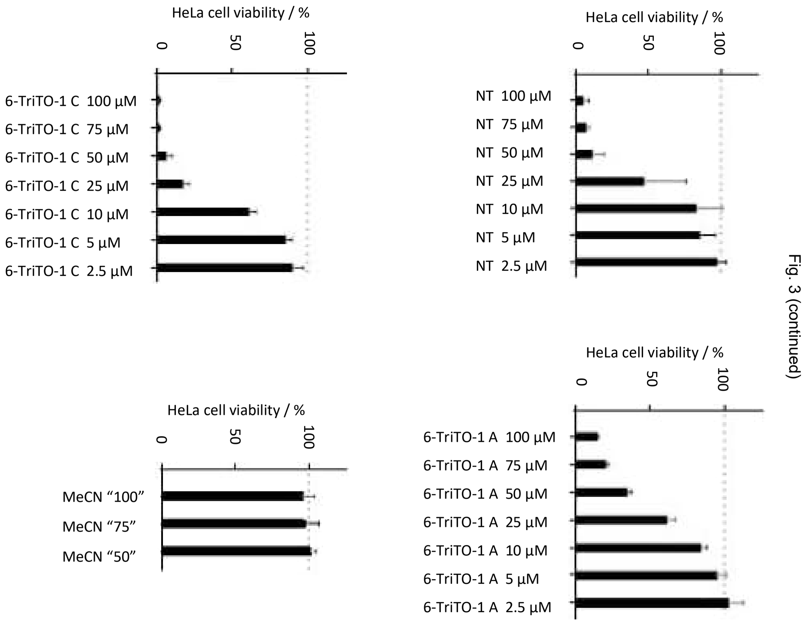

The triazole amine derivatives 6-TramTO-1 (**1**), 6-TramTAS (**2**) and 6-TramTO-3 (**3**) do not interfere with the growth of Escherichia coli (E. coli) NEB5α (New England Biolabs), while the previously described dyes, without the triazole amine side chain (**4-6**) do. Even at 5 µM concentration no toxicity is observed regarding compounds **1-3,** clearly proving that TramTOs and TramTAS (**1-3**) are non-toxic to microorganisms (cf. Fig. 2). Fig. 3 exemplarily shows the viability of HeLa cells upon staining with cyanine dyes. HeLa cells are eukaryotic cells, therefore these experiments show the widespread applicability of the invention.

### Experimental procedure for measuring of cell viability

HeLa cells are grown in a humidified cell-culture incubator (New Brunswick Scientific, USA) at 37 °C under CO₂ (g) (5%, v/v). Growth medium is DMEM nutrient medium supplemented with fetal bovine serum (FBS) (10%, v/v), penicillin (100 units/mL), and streptomycin (100 µg/mL). HeLa cells (2×10⁴) are seeded into black 96-well microtiter plates (Greiner Bio-One, Austria) in DMEM (200 µL, 2.5% FBS). For assessing the viability stocks of the compounds of interest in milliQ water are added at different concentrations and incubated for 24 h. Resazurin fluorescence-based cell viability assay is performed to evaluate cell viability.

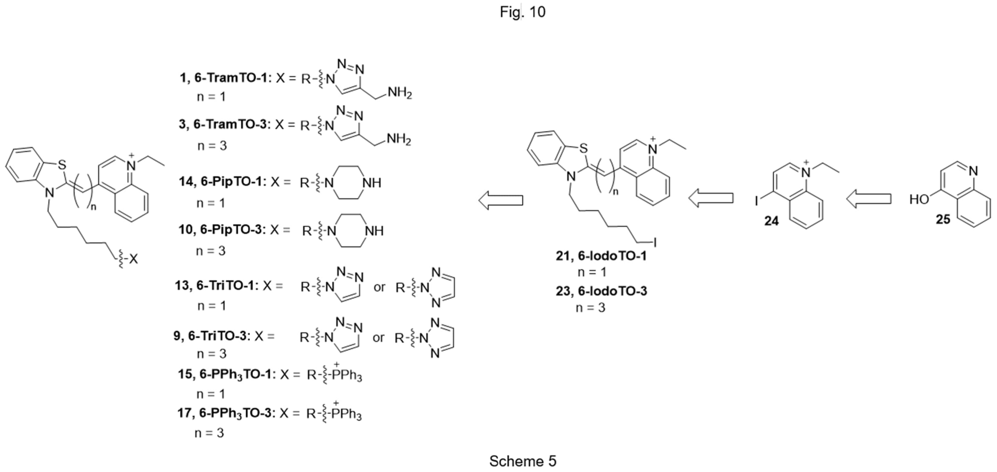

Measurements of cell viability is exemplarily performed with compounds 18, 19 and 20: HeLa cells are treated with different concentrations (10 µM, 7.5 µM, 5 µM, 2.5 µM, 1 µM and 0.5 µM) of the dyes (18, 19, 20) in triplicated. Viability is then examined after 21h incubation time by resazurin-based assay. The results of the tests are depicted in Figs. 10A and 10B.

### Experimental procedure for measuring bacterial growth curves

*E. coli* NEB5α are cultured in lysogeny broth (LB) liquid medium and incubated at 37 °C, 200 rpm. In clear 96-well microtiter plates (Nunc, USA) in a final volume of 250 µL 5 µL of cell suspension at exponential growth phase were added and stocks of the compounds of interest in milliQ water, DMSO or MeCN (final vehicle concentration 0.8%, v/v). Bacterial growth is monitored at OD₆₀₀, utilizing a microplate reader (Tecan, Switzerland).

### Fluorescence titrations

The affinity of the TramTO derivatives towards dsDNA is usually higher than the affinity of the derivatives without the modified side chain according to this invention. Usually the TramTOs according to the invention were the stronger binders compared to the corresponding controls (**4-6**) with the similar fluorescence, however the green control CH₃TO-1 (4) is a stronger binder then the 6-TramTAS, the 6-TramTO-1 is superior to 6-TramTO-3 (about x2) and 6-TramTAS (about x3). *K*_{D} values are determined as is well known to any person being of ordinary skill in the art. Table 2 shows the results obtained.

**Table 2: Exemplary results of fluorescence titrations (affinity towards dsDNA)**

| | RFU -/+ DNA | Slits (nm) | *K*_{D} |
|---|---|---|---|
| 6-TramTO-1 (**1**) | 1.2/460 | 5/5 | 8.63 ± 4.34 µM |
| CH₃TO-1 (**4**) | 0.2/125 | 10/10 (for 5/5: ~10 RFU) | 20.56 ± 6.67 µM |
| 6-TramTAS (**2**) | 2.9/90 | 10/10 (for 5/5: ~8 RFU) | 30.09 ± 4.61 µM or 28.07 ± 2.33 µM |
| CH₃TAS (**5**) | 2.3/33 | 10/10 (for 5/5: ~2 RFU) | Not calculable |
| 6-TramTO-3 (**3**) | 11.8/280 | 5/5 | 22.96 ± 1.81 µM / 19.5±2.6 µM (literature value) |
| CH₃TO-3 (**6**) | 6.8/210 | 5/5 | 44.98 ± 1.05 µM |

### Flow Cytometry

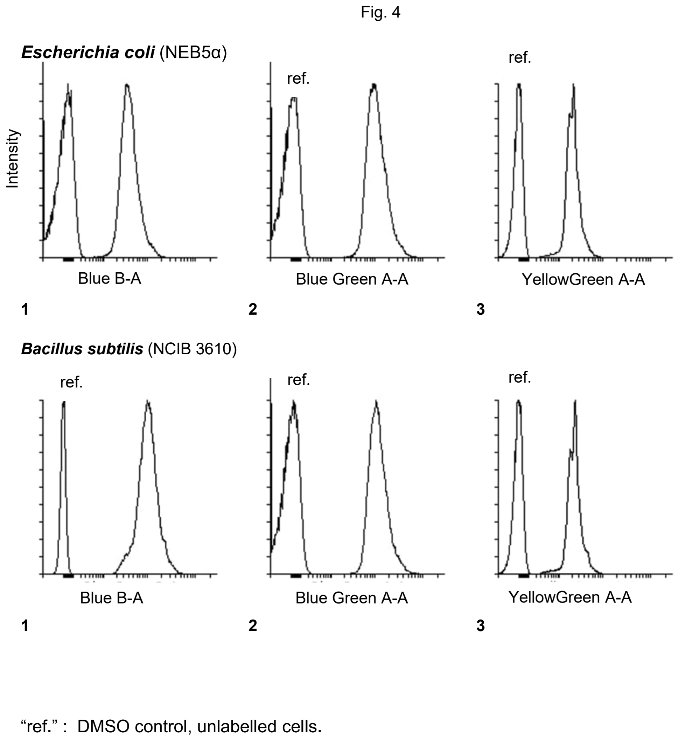

The flow cytometric measurements are performed by use of the LSR Fortessa cell analyser (BD Biosciences, USA) or other commercially available apparatuses (e.g. FACSAria III (BD Biosciences, USA) and Guava easyCyte (Merck Millipore, USA)). Any person of ordinary skill in the art knows how to adjust the different apparatuses commercially available in order to perform comparable experiments exerting the experimental parameters described herein. A minimum of 10000 events per sample is analysed. The measurements are each performed in at least two independent experiments. Bacteria are labelled for 15 min with 5 µM of the corresponding dyes. All dyes showed > 99.9% labelling. Fig. 4 shows graphs of the results, exemplarily obtained with *E. Coli* (NEB5α) and *B*. *Subtilis* (NCIB 3610).

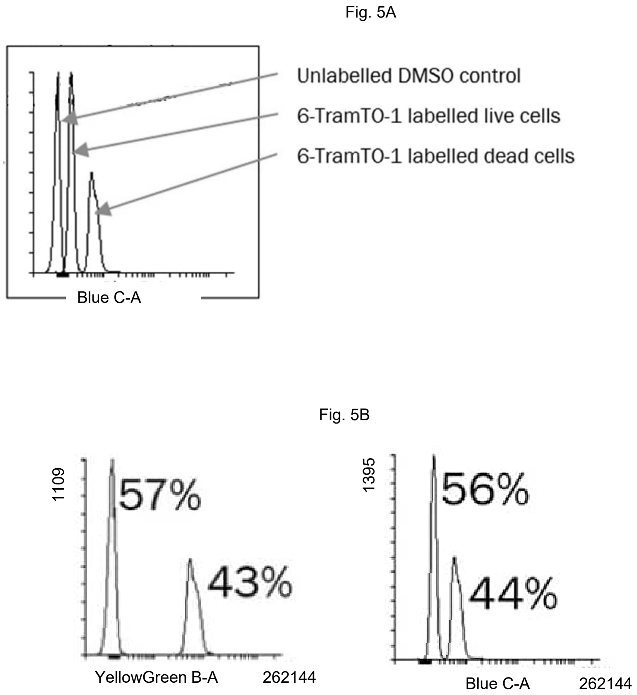

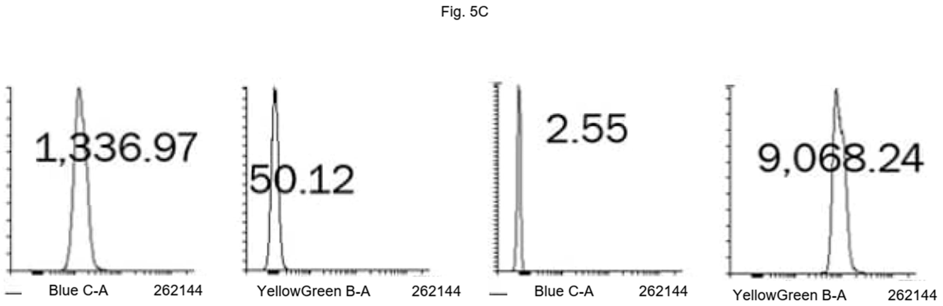

For 6-TramTO-1 (1) one can see clear labelling when 5 µM of the dye are added for only 5 min before the measurements, dead cells (treated with 70% isopropanol for 1h, then washed with saline 3x) being labelled about 10x stronger: MFI 135 vs 1337. Figs. 5A - 5C show additional data.

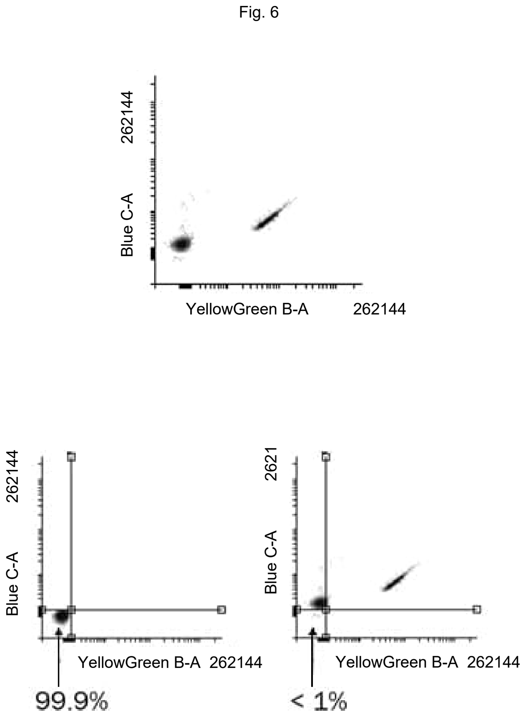

As can obviously be seen from the data shown in Fig. 6, co-staining with PI is not necessary upon usage of the inventive cyanine dyes in order to distinguish live from dead cells. The green (Blue C) channel gave the same results than the orange/ PI channel (YellowGreen B) in comparison with commercially available Kit BacLight^{™} of ThermoFisher.

### Dual and triple labelling

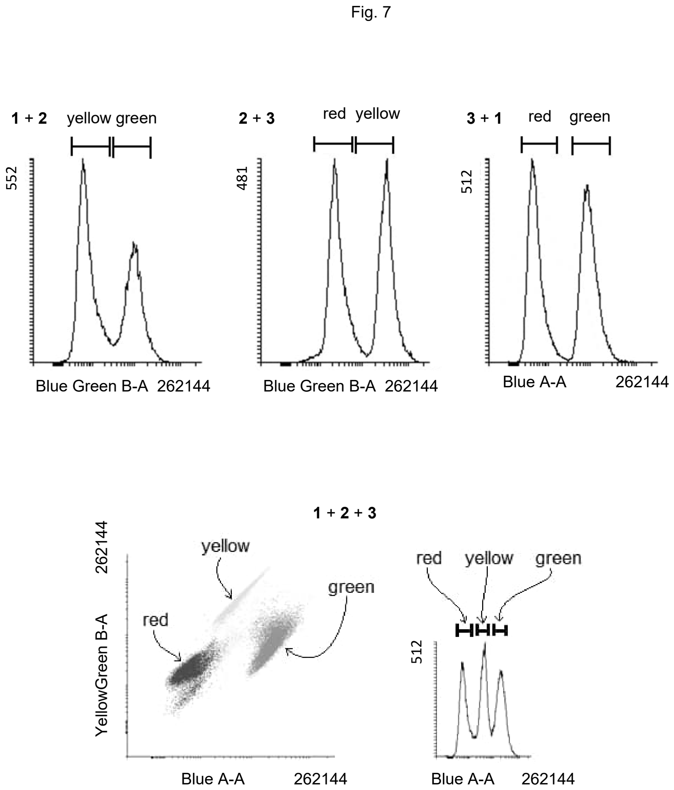

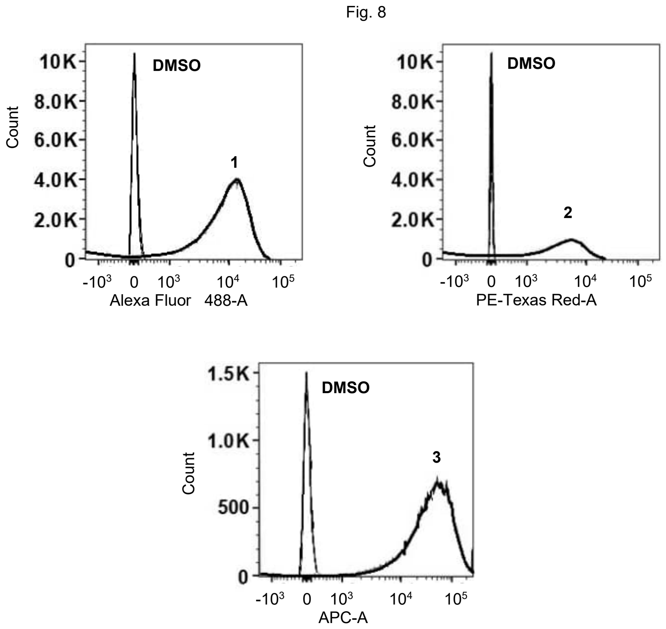

Experimental data shows that dual and even triple labelling is also possible with the TramTO dyes (examples presented in Fig. 7 using *Bacillus subtilis* and in Fig. 8 using *Klebsiella pneumoniae*).

### Exemplary Phagocytosis studies with different bacterial strains exemplarily labelled with 1 or 3

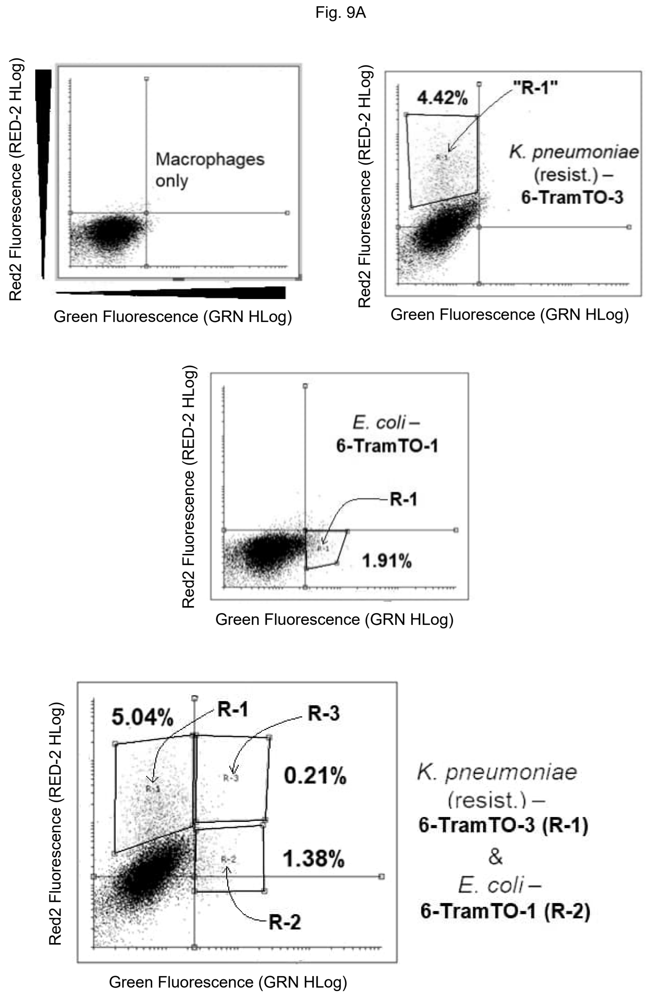

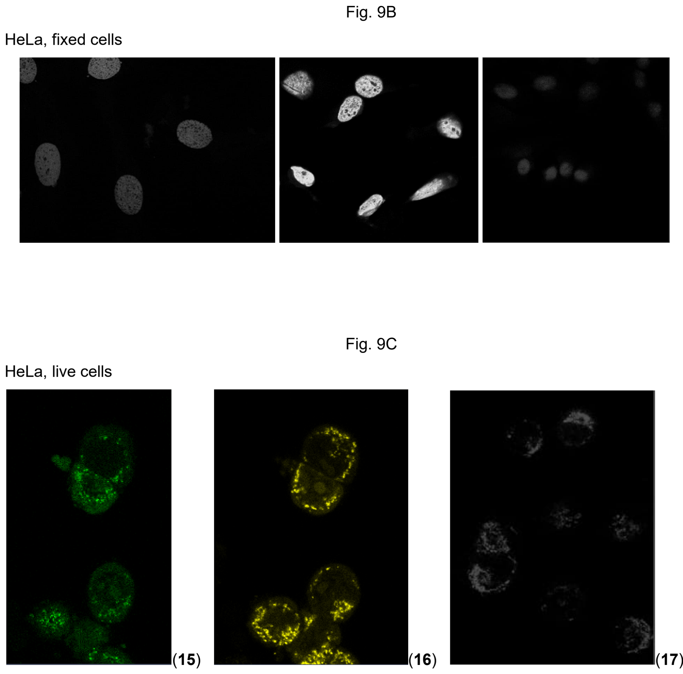

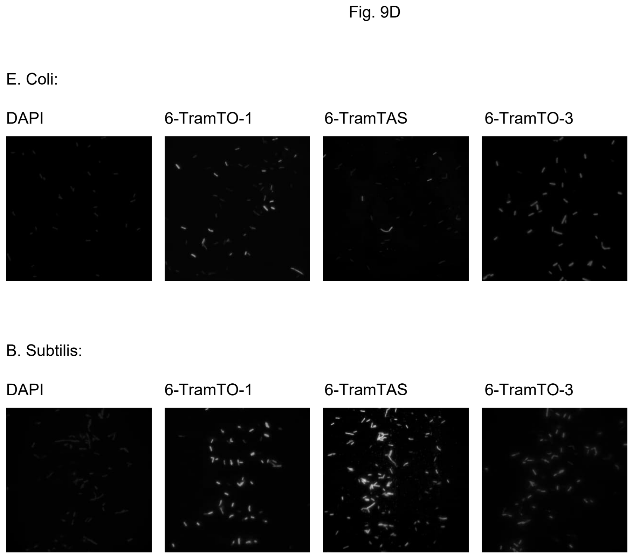

The applicability of the invention is proven by exemplary Phagocytosis studies as is described below (cf. Fig. 9A, Fig. 9B and Fig. 9C):
FACS data is recorded using a Guava easyCyte flow cytometer (Millipore). Bacteria are labelled for 15 min with 5 µM of the corresponding dye, washed 3 times with DPBS. After the last wash bacteria are brought to an OD₆₀₀ nm of 2.0 and 2.3 µL in such a way that an equivalent of bacteria are added to each well to achieve a multiplicity of infection (MOI) of 5 (MOI = 5 + 5 for experiments with two bacterial strains). Culture plates are then centrifuged for 10 min at 250 g and 37°C to establish physical contact between macrophages and bacteria. Subsequently, culture plates are transferred back into a 37°C incubator with CO₂ atmosphere for 50 min. After the stimulation period the cells are washed once with DPBS prior to flow cytometry. Macrophages are detached carefully from the culture dishes by using a rubber scraper.

### Nuclear Magnetic Resonance Spectroscopy (NMR)

All NMR spectra are automatically measured at 300 K either in a Bruker AV III HD 300 MHz at a frequency of 300 MHz (¹H) or 75 MHz (¹³C) or in a Bruker AV III HD 500 MHz at a frequency of 500 MHz (¹H) or 125 MHz (¹³C). All chemical shifts (δ) are relative to tetramethylsilane (TMS), i.e. δ(TMS) = 0 ppm. As internal standards, deuterated chloroform (CDCl₃), dimethyl sulfoxide (DMSO) with TMS are used. Solvent shifts (ppm): δ(CHCl₃) = 7.26 ppm (¹H), δ(CHCl₃) = 77.16 ppm (¹³C), δ(DMSO) = 2.50 ppm (¹H), δ(DMSO) = 39.52 ppm (¹³C). The assignment of each signal is based on two-dimensional nuclear magnetic resonance spectroscopy (2D NMR), i.e. heteronuclear single quantum coherence (HSQC), heteronuclear multiple bond correlation spectroscopy (HMBC) and correlation spectroscopy (COSY).

### Mass Spectrometry

Electrospray ionization mass spectrometry (ESI-MS) is performed on a Finnigan LTQ-FT(Thermo Fischer Scientific). EI-MS is performed on an AccuTOF GCv (JEOL).

### Analytic HPLC

Characterization is performed via analytical RP-HPLC-MS on an Agilent 1260 Infinity II HPLC-System (Agilent Technologies). Agilent eclipse XDB-C18 column (5 µm, 4.6 × 150 mm) using isocratic regime during the first 5 min, followed by a linear gradient over 30 min of solvent B as stated below with a flow rate of 0.2 mL/min at 55 °C. The detection was carried out by monitoring the absorbance at 220 nm. The eluents are ultrapure H₂O (solvent A) with addition of 0.05% TFA and MeCN (solvent B) with addition of 0.03% TFA.
Gradient 1: 5% to 75% of solvent B
Gradient 2: 5% to 95% of solvent B

### Synthetic Procedures

Scheme 5 (Fig. 10) shows the general retrosynthetic pathway of the production of the cyanine dyes according to the invention.

### Synthesis of 6-TramTO-1

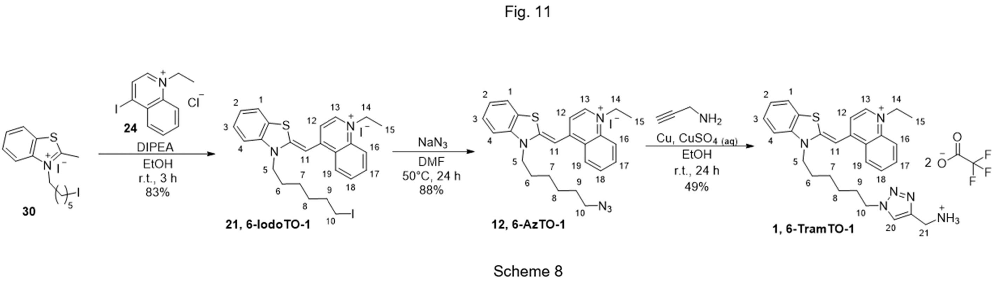

This synthesis is based on the scaffold of thiazole orange (TO), known within the state of the art, but proceeding beyond the state of the art (cf. Fig. 11, scheme 8).

**(Z)-1-ethyl-4-((3-(6-iodohexyl)benzo[d]thiazol-2(3H)-ylidene)methyl)quinolin-1-ium (6-IodoTO-1, 21):** Benzothiazolium (**30,** 521 mg, 1.07 mmol) and quinolinium (**24,** 342 mg, 1.07 mmol) are suspended in EtOH (HPLC-grade, 16.0 mL) and DIPEA (410 µL, 2.35 mmol) added. The solution is stirred in the dark at r.t. for 1 h, the solvent removed and the crude purified by flash column chromatography (allox, MeOH in CH₂Cl₂: 0% → 0.2% → 0.5%→ 0.7%) to yield the desired product (**21**, 359 mg, 0.56 mmol, 52%) as red solid. **R*_{f}*** = 0.45 (CH₂Cl₂/MeOH 10:1). **¹H-NMR** (500 MHz, DMSO-*d*₆, δ): 8.75 (d, 1H, *J* = 8.7 Hz, H-16), 8.67 (d, 1H, *J* = 7.3 Hz, H-13), 8.16 (d, 1H, *J =* 8.6 Hz, H-19), 8.05 (d, 1H, *J =* 7.2 Hz, H-4), 8.03 - 7.99 (m, 1H, H-17), 7.81 - 7.76 (m, 2H, H-1, H-18), 7.63 - 7.59 (m, 1H, H-2), 7.44 - 7.39 (m, 2H, H-3, H-12), 6.93 (s, 1H, H-11), 4.70 - 4.60 (m, 4H, H-14, H-5), 3.24 (t, 2H, *J* = 6.9 Hz, H-10), 1.83 - 1.76 (m, 2H, H-9), 1.76 - 1.70 (m, 2H, H-6), 1.50 - 1.45 (m, 5H, H-8, H-15), 1.45 - 1.39 (m, 2H, H-7). **¹³C-NMR** (125 MHz, DMSO-*d*₆, δ): 159.4 (C_{q}), 148.7 (C_{q}), 144.0 (CH), 140.0 (C_{q}), 136.9 (C_{q}), 133.3 (CH), 128.3 (CH), 127.0 (CH), 125.7 (CH), 124.6 (CH), 124.3 (C_{q}), 123.9 (C_{q}), 123.0 (CH), 118.1 (CH), 113.0 (CH), 108.3 (CH), 87.7 (CH), 49.5 (CH₂), 45.6 (CH₂), 32.7 (CH₂), 29.6 (CH₂), 26.7 (CH₂), 24.9 (CH₂), 14.8 (CH₃), 8.8 (CH₂). **HRMS-ESI⁺** (*m*/*z*): [M]⁺ calcd for C₂₅H₂₈IN₂S⁺, 515.1012; found, 515.1023.

**(Z)-1-ethyl-4-((3-(6-iodohexyl)benzo[d]thiazol-2(3H)-ylidene)methyl)quinolin-1-ium (6-AzTO-1, 12):** lodo derivative **6-IodoTO-1** (**5**, 13.1 mg, 23.8 µmol) and NaN₃ (2.00 mg, 30.9 µmol) are dissolved in DMF (500 µL) and stirred for 25 h at 50 °C in the dark. The solvent is removed and the crude purified by flash column chromatography (allox basic, MeOH in CH₂Cl₂: 0% → 2.0%) to yield the desired product (**12,** 10.8 mg, 19.9 µmol, 84%) as red solid. ***R_{f}*** = 0.43 (CH₂Cl₂/MeOH 10:1). **¹H-NMR** (500 MHz, DMSO-*d*₆, δ): 8.76 (d, 1H, ³*J* = 8.4 Hz, H-9), 8.67 (d, 1H, ³*J* = 7.2 Hz, H-10), 8.17 (d, 1H, ³*J* = 8.6 Hz, H-6), 8.06 (dd, 1H, ³*J* = 7.9 Hz, ⁴*J* = 1.0 Hz, H-4), 8.03- 7.99 (m, 1H, H-8), 7.81- 7.76 (m, 2H, H-1, H-7), 7.62 (td, 1H, ³*J =* 7.8 Hz, ⁴*J* = 1.1 Hz, H-2), 7.45- 7.40 (m, 2H, H-3, H-11), 6.94 (s, 1H, H-5), 4.69- 4.62 (m, 4H, H-12, H-14), 3.32- 3.27 (m, 2H, H-19), 1.84- 1.77 (m, 2H, H-15), 1.54- 1.44 (m, 9H, H-13, H-16, H-17, H-18) ppm.**¹³C-NMR** (75 MHz, DMSO-*d₆, δ*): 159.4 (Cq), 148.7 (Cq), 144.1 (C-10), 140.0 (Cq), 136.9 (Cq), 133.4 (C-8), 128.3 (C-2), 126.9 (C-7), 125.8 (C-9), 124.6 (C-3), 124.3 (Cq), 123.9 (Cq), 123.0 (C-4), 118.1 (C-6), 113.1 (C-1), 108.3 (C-11), 87.7 (C-5), 50.5 (C-19), 49.5 (C-12), 45.6 (C-14), 28.1 (C-18), 26.8 (C-15), 25.9 (C-17), 25.5 (C-16), 14.8 (C-13) ppm. **HRMS-ESI⁺** *(m*/*z):* [M]⁺ calcd for C₂₅H₂₈N₅S⁺, 430.2060; found, 430.2063.

**(Z)-4-((3-(6-(4-(ammoniomethyl)-1H-1,2,3-triazol-1-yl)hexyl)benzo[d]thiazol-2(3H)-ylidene)methyl)-1-ethylquinolin-1-ium (6-TramTO-1, 1):** Under inert atmosphere, azido derivative **6-AzTO-1** (**12**, 60.0 mg, 108 µmol) is dissolved in ethanol (1.13 mL) and propargylamine (14.2 µL, 222 µmol), copper powder (8.12 mg, 128 µmol) and copper sulfate (100 mM, 53.8 µL, 5.38 µmol) are added. The resulting mixture is stirred for 20 h, filtered and the solvent removed. The product is purified by preparative HPLC (Gilson 5-75%) to yield the desired product (**1**, 38.0 mg, 53.3 µmol, 50%) as red solid. **R*ₜ*** = 20.2 min. **¹H-NMR** (500 MHz, DMSO-*d*₆, δ): 8.75 (d, 1H, *J* = 8.8 Hz, H-16), 8.67 (d, 1H, *J* = 7.2 Hz, H-13), 8.29 (br s, 3H, NH₃⁺), 8.17 (d, 1H, *J* = 8.8 Hz, H-19), 8.10 (s, 1H, H-20), 8.05 (dd, 1H, *J* = 7.9 Hz, *J =* 0.9 Hz, H-4), 8.03 - 7.99 (m, 1H, H-17), 7.80 - 7.75 (m, 2H, H-1, H-18), 7.64 - 7.59 (m, 1H, H-2), 7.45 - 7.41 (m, 2H, H-3, H-12), 6.94 (s, 1H, H-11), 4.66 (q, 2H, *J =* 7.2 Hz, H-14), 4.63 (t, 2H, *J =* 7.4 Hz, H-5), 4.37 (t, 2H, *J* = 7.0 Hz, H-10), 4.12 - 4.06 (m, 2H, H-21), 1.83 - 1.75 (m, 4H, H-6, H-9), 1.54 - 1.46 (m, 5H, H-8, H-15), 1.36 - 1.28 (m, 2H, H-7). **¹³C-NMR** (125 MHz, DMSO-*d*₆, δ): 159.4 (C_{q}), 148.7 (C_{q}), 144.0 (CH), 140.0 (C_{q}), 136.9 (C_{q}), 133.3 (CH), 128.2 (CH), 126.9 (CH), 125.7 (CH), 124.5 (CH), 124.3 (C_{q}), 124.1 (2C, CH, C_{q}), 123.9 (C_{q}), 123.0 (CH), 118.0 (CH), 113.0 (CH), 108.3 (CH), 87.6 (CH), 49.5 (CH₂), 49.3 (CH₂), 45.6 (CH₂), 33.9 (CH₂), 29.6 (CH₂), 26.8 (CH₂), 25.6 (CH₂), 25.4 (CH₂), 14.7 (CH₃). **HRMS-ESI⁺** *(m*/*z):* [M]⁺ calcd for C₂₈H₃₃N₆S⁺, 485.2482; found, 485.2487.

### Synthesis of 6-xxxTO-1 and of 6-xxxTO-3

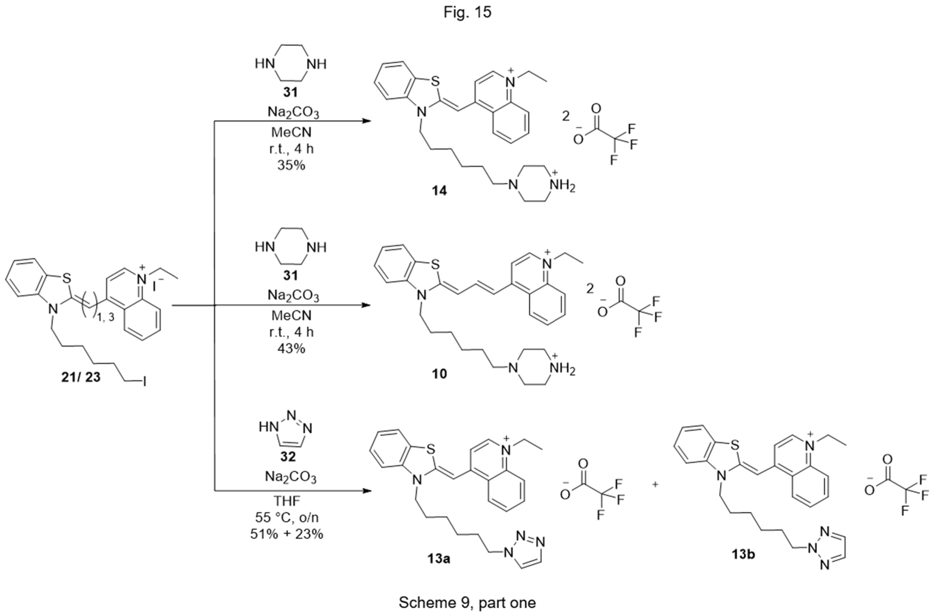

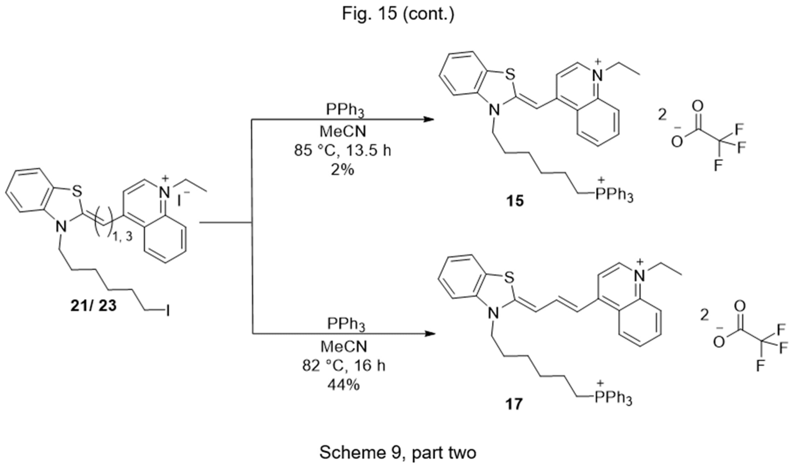

This synthesis is based on the nucleophilic substitution of the iodo substituent of 6-IodoTO-1 and 6-lodoTO-3 (cf. Fig. 15, scheme 9).

**(Z)-1-ethyl-4-((3-(6-(piperazin-1-ium-1-yl)hexyl)benzo[d]thiazol-2(3H)-ylidene)methyl)quinolin-1-ium (6-PipTO-1, 14):** lodo derivative **6-IodoTO-1** (**21**, 100 mg, 156 µmol) and Na₂CO₃ (24.5 mg, 231 µmol) are suspended in MeCN (320 µL) and piperazine (48.9 µL, 624 µmol) added. The reaction mixture is stirred for 4 h at r.t., water added and the product extracted with CH₂Cl₂ and dried over MgSO₄. The product is purified by preparative HPLC (Gilson 5-75%) to yield the desired product (**14,** 38.3 mg, 54.7 µmol, 35%) as red solid. **R*ₜ*** = 18.0 min. **¹H-NMR** (500 MHz, DMSO-*d*₆, δ): 9.32 (br s, 2H, NH₂⁺), 8.76 (d, 1H, *J =* 8.0 Hz, H-16), 8.67 (d, 1H, *J =* 7.3 Hz, H-13), 8.17 (d, 1H, *J =* 8.6 Hz, H-19), 8.06 (dd, 1H, *J* = 7.8 Hz, *J =* 0.6 Hz, H-4), 8.03 - 7.99 (m, 1H, H-17), 7.80 - 7.75 (m, 2H, H-1, H-18), 7.64 - 7.59 (m, 1H, H-2), 7.46 - 7.41 (m, 2H, H-3, H-12), 6.95 (s, 1H, H-11), 4.69 - 4.62 (m, 4H, H-14, H-5), 3.49 - 3.09 (m, 8H, H-20, H-20', H-21, H-21'), 3.05 - 2.95 (m, 2H, H-10), 1.86 - 1.77 (m, 2H, H-9), 1.63 - 1.55 (m, 2H, H-6), 1.52 - 1.45 (m, 5H, H-8, H-15), 1.40 - 1.33 (m, 2H, H-7). **¹³C-NMR** (125 MHz, DMSO-*d₆*, δ): 159.4 (C_{q}), 148.8 (C_{q}), 144.0 (CH), 140.0 (C_{q}), 136.9 (C_{q}), 133.3 (CH), 128.2 (CH), 126.9 (CH), 125.7 (CH), 124.5 (CH), 124.3 (C_{q}), 123.9 (C_{q}), 123.0 (CH), 118.0 (CH), 113.0 (CH), 108.3 (CH), 87.6 (CH), 55.8 (CH₂), 49.5 (CH₂), 48.1 (CH₂), 45.6 (CH₂), 40.5 (CH₂), 26.8 (CH₂), 23.3 (CH₂), 25.8 (CH₂), 25.6 (CH₂), 14.7 (CH₃). **HRMS-ESI⁺** (m/z): [M]⁺ calcd for C₂₉H₃₇N₄S⁺, 473.2733; found, 473.2731.

**1-ethyl-4-((1E,3Z)-3-(3-(6-(piperazin-1-yl)hexyl)benzo[d]thiazol-2(3H)-ylidene)prop-1-en-1-yl)quinolin-1-ium (6-PipTO-3, 10):** The iodo derivate **23**(100 mg, 150 µmol, 1.00 eq) and Na₂CO₃ (24.5 mg, 231 µmol, 1.54 eq) are suspended in acetonitrile (320 µL) and piperazine (53.8 mg, 624 mmol, 4.16 eq) is added. The reaction mixture is stirred for 6 h at room temperature. Distilled water (1.0 mL) is added, and the product is extracted with CH₂Cl₂ and dried over MgSO₄. The solvent is evaporated under reduced pressure, and the crude is purified using a Büchi pure system with MeCN in H₂O (0 % → 45 %) as a solvent to yield the desired product **12** (40.3 mg, 64.3 µmol, 43 %) as a blue solid.**¹H-NMR** (500 MHz, DMSO-*d₆*, δ): 8.51- 8.44 (m, 2H, H-11, H-12), 8.16 (t, 1H, ³*J* = 12.7 Hz, H-6), 8.12- 8.08 (m, 1H, H-8), 7.97 (td, 1H, ³*J* = 7.9 Hz, ⁴*J* = 1.3 Hz, H-10), 7.91- 7.87 (m, 2H, H-4, H-13), 7.74- 7.69 (m, 1H, H-9), 7.63- 7.58 (m, 1H, H-1), td (7.49, 1H, ³*J* = 7.8 Hz, ⁴*J* = 1.1 Hz, H-3), 7.34- 7.27 (m, 1H, H-2), 7.19- 7.13 (m, 1H, H-7), 6.53 (d, 1H, ³*J* = 12.2 Hz, H-5), 4.61 (q, 2H, ³*J* = 7.2 Hz, H-14), 4.26-4.21 (m, 2H, H-16), 3.14-2.99 (m, 3H, H-21), 1.78- 1.70 (m, 2H, H-20), 1.70- 1.57 (m, 2H, H-17), 1.49- 1.42 (m, 4H, H-15, H-19), 1.41- 1.35 (m, 2H, H-18) ppm.**¹³C-NMR** (75 MHz, DMSO-*d*₆, δ): 160.8 (Cq), 158.4 (Cq), 158.1 (Cq), 150.5 (Cq), 141.5 (Cq), 137.8 (Cq), 144.0 (C-8), 142.3 (C-12), 133.5 (C-10), 127.7 (C-3), 126.7 (C-9), 125.3 (C-11), 124.3 (C-2), 122.6 (C-13), 118.1 (C-6), 112.5 (C-1), 110.1 (C-4), 109.6 (C-7), 98.5 (C-5), 55.7 (C-22, C-26), 49.4 (C-14), 47.9 (C-23, C-25), 45.5 (C-16), 26.9 (C-19), 25.8 (C-18), 25.7 (C-17), 14.8 (C-15) ppm.**HRMS-ESI⁺** (*m*/*z*): [M+2H]⁺ calcd. for C₃₁H₃₉N₄S⁺ 499.2890; found 501.2684.

**(Z)-4-((3-(6-(1H-1,2,3-triazol-1-yl)hexyl)benzo[d]thiazol-2(3H)-ylidene)methyl)-1-ethylquinolin-1-ium (6-Tri_{(asym.)}TO-1, 13a) and (Z)-4-((3-(6-(2H-1,2,3-triazol-2-yl)hexyl)benzo[d]thiazol-2(3H)-ylidene)methyl)-1-ethylquinolin-1-ium (6-Tri_{(sym.)}TO-1, 13b):**
lodo derivative **6-IodoTO-1** (**21**, 150.0 mg, 0.23 mmol) and sodium carbonate (99.0 mg, 0.93 mmol) are dissolved in THF (467 µL) and 1-H-1,2,3-triazole (**16**, 83.1 µL, 0.47 mmol) is added. The reaction mixture is stirred in the dark for 3 h at 55 °C. 1-*H*-1,2,3-triazole (**16,** 83.1 µL, 0.47 mmol) is added and the mixture is stirred overnight under the same conditions. The solution is washed with distilled water. The aqueous layer is extracted with CH₂Cl₂ and the organic phase is dried over magnesium sulfate. The crude products are purified by preparative HPLC (Gilson 5-75%) to yield **6-Tri_{(asym.)}TO-1** (**13a,** 69.6 mg, 0.12 mmol, 51%) and **6-Tri_{(sym.)}TO-1** (**13b,** 31.7 mg, 0.05 mmol, 23%) as pink solids.

### 6-Tri_{(asym.)}TO-1 (13a):

**R*ₜ*** = 21.6 min (gradient 2). **¹H-NMR** (300 MHz, DMSO-*d₆*, δ): 8.73 (d, 1H, *J* = 8.5 Hz, H-16), 8.66 (d, 1H, *J* = 7.2 Hz, H-13), 8.17 (d, 1H, *J* = 8.6 Hz, H-19), 8.10 - 7.97 (m, 3H, H-4, H-17, H-20), 7.82 - 7.74 (m, 2H, H-1, H-18), 7.67 (s, 1H, H-21), 7.65 - 7.58 (m, 1H, H-2), 7.47 - 7.39 (m, 2H, H-3, H-12), 6.93 (s, 1H, H-11), 4.70 - 4.59 (m, 4H, H-14, H-5), 4.35 (m, 2H, *J* = 7.0 Hz, H-10), 1.87 - 1.74 (m, 4H, H-9, H-6), 1.54 - 1.43 (m, 5H, H-8, H-15), 1.36 - 1.26 (m, 2H, H-7). **¹³C-NMR** (75 MHz, DMSO-*d*₆, δ): 159.4 (C_{q}), 148.7 (C_{q}), 144.0 (CH), 140.0 (C_{q}), 136.8 (C_{q}), 133.3 (CH), 133.1 (CH), 128.2 (CH), 126.9 (CH), 125.7 (CH), 124.5 (2C, CH), 124.3 (C_{q}), 123.9 (C_{q}), 122.9 (CH), 118.0 (CH), 112.9 (CH), 108.3 (CH), 87.6 (CH), 49.5 (CH₂), 49.0 (CH₂), 45.6 (CH₂), 29.5 (CH₂), 26.7 (CH₂), 25.6 (CH₂), 25.4 (CH₂), 14.6 (CH₃). **HRMS-ESI⁺** *(m*/*z):* [M]⁺ calcd for C₂₇H₃₀N₅S⁺, 456.2216; found, 456.2212.

### 6-Tri_{(sym.)}TO-1 (13b):

**R*ₜ*** = 23.6 min (gradient 2). **¹H-NMR** (300 MHz, DMSO-*d*₆, δ): 8.72 (d, 1H, *J* = 8.2 Hz, H-16), 8.66 (d, 1H, *J* = 7.2 Hz, H-13), 8.16 (d, 1H, *J* = 8.6 Hz, H-19), 8.07 - 7.97 (m, 2H, H-4, H-17), 7.80 - 7.73 (m, 2H, H-1, H-18), 7.70 (s, 2H, H-20, H-20'), 7.64 - 7.57 (m, 1H, H-2), 7.45 - 7.38 (m, 2H, H-3, H-12), 6.91 (s, 1H, H-11), 4.70 - 4.56 (m, 4H, H-14, H-5), 4.38 (m, 2H, *J* = 6.9 Hz, H-10), 1.88 - 1.73 (m, 4H, H-9, H-6), 1.52 - 1.42 (m, 5H, H-8, H-15), 1.34 - 1.26 (m, 2H, H-7). **¹³C-NMR** (75 MHz, DMSO-*d*₆, δ): 159.4 (C_{q}), 148.7 (C_{q}), 144.0 (CH), 140.0 (C_{q}), 136.9 (C_{q}), 134.0 (2C, CH), 133.3 (CH), 128.2 (CH), 126.9 (CH), 125.7 (CH), 124.5 (CH), 124.3 (C_{q}), 123.9 (C_{q}), 122.9 (CH), 118.0 (CH), 112.9 (CH), 108.3 (CH), 87.6 (CH), 53.8 (CH₂), 49.4 (CH₂), 45.6 (CH₂), 28.9 (CH₂), 26.7 (CH₂), 25.6 (CH₂), 25.3 (CH₂), 14.6 (CH₃). **HRMS-ESI⁺** *(m*/*z):* [M]⁺ calcd for C₂₇H₃₀N₅S⁺, 456.2216; found, 456.2213.

**(Z)-1-ethyl-4-((3-(6-(triphenylphosphonio)hexyl)benzo[d]thiazol-2(3H)-ylidene)methyl)quinolin-1-ium (6-PPh₃TO-1, 15):** lodo derivative **6-IodoTO-1** (**21,** 118 mg, 184 µmol) and PPh₃ (145 mg, 551 µmol) are suspended in MeCN (3.00 mL) and stirred for1 h at r.t. and 13.5 h at 85 °C. The solvent is removed and the crude filtered over whool. The product is purified by preparative HPLC (Gilson 5-75%) to yield the desired product (**15, 3.50** mg, 3.88 µmol, 2%) as red solid. **R*ₜ*** = 25.1 min (gradient 1). **HRMS-ESI⁺** (*m*/*z*): [M]²⁺ calcd for C₄₃H₄₃N₂PS²⁺, 325.1437; found, 325.1437.

**1-ethyl-4-((1E,3Z)-3-(3-(6-(triphenylphosphonio)hexyl)benzo[d]thiazol-2(3H)-ylidene)prop-1-en-1-yl)quinolin-1-ium (6-PPh₃TO-3, 17):** derivative **6-lodoTO-3** (**23,** 49.9 mg, 74.7 µmol) and PPh₃ (58.9 mg, 224 µmol) are suspended in MeCN (3.00 mL) and stirred 16 h at 82 °C. The solvent is removed and the crude filtered over whool. The product is purified by preparative HPLC (Gilson 5-75%) to yield the desired product **(**17, 1.90 mg, 2.10 µmol, 44%) as blue solid. **R*ₜ*** = 26.9 min (gradient 1); **¹H-NMR (500 MHz, DMSO-*d*₆)** δ = 8.45 (d, *³J_{H-H} =* 7.1 Hz, 1H, H23), 8.42 (d, *³J_{H-H} =* 8.2 Hz, 1H, H26), 8.14 (t, *³J_{H-H} =* 12.8 Hz, 1H, H18), 8.09 (d, *³J_{H-H} =* 8.8 Hz, 1H, H29), 7.95 (t, *³J_{H-H} =* 7.3 Hz, 1H, H31), 7.87 (m, 5H, H3, H24, H37, H42, H47), 7.76 (m, 12H, H35, H36, H38 - 41, H43 - 46, H48, H49), 7.67 (t, *³J_{H-H} =* 7.7 Hz, 1H, H30), 7.56 (d, *³J_{H-H} =* 8.3 Hz, 1H, H6), 7.46 (t, *³ J_{H-H} =* 7.3 Hz, 1H, H2), 7.30 (t, *³J_{H-H} =* 7.8 Hz, 1H, H1), 7.11 *(d, ³J_{H-H} =* 13.3 Hz, 1H, H19), 6.50 (d, *³ J_{H-H} =* 12.3 Hz, 1H, H10), 4.61 (q, *³J_{H-H} =* 7.1 Hz, 2H, H21), 4.19 (t, *³J_{H-H} =* 7.4 Hz, 2H, H11), 3.59 (m, 2H, H16), 1.68 (m, 2H, H12), 1.59 (s, 4H, H13, H14), 1.34 (t, *³J_{H-H} =* 7.1 Hz, 5H, H15, H20) ppm; **¹³C-NMR (126 MHz, DMSO-*d*₆)** *δ* = 160.9 (s, 1C, C25), 150.4 (s, 1C, C8), 143.9 (s, 1C, C18), 142.3 (s, 1C, C23), 141.5 (s, 1C, C27), 137.7 (s, 1C, C4), 134.9 (s, 3C, C37, C42, C47), 133.6 (d, 6C, C35, C39, C40, C44, C45, C49), 133.5 (s, 1C, C31), 130.3 (d, 3C, C36, C38, C41, C43, C46, C48), 127.7 (s, 1C, C2), 126.7 (s, 1C, C30), 125.2 (s, 1C, C26), 124.8 (s, 1C, C1), 124.3 (s, 1C, C5), 122.7 (s, 1C, C24), 118.9 (s, 3C, C32-34), 118.2 (s, 1C, C29), 112.4 (s, 1C, C6), 110.0 (s, 1C, C3), 109.5 (s, 1C, C19), 98.5 (s, 1C, C10), 49.4 (s, 1C, C21), 45.4 (s, 1C, C11), 29.4 (s, 1C, C13), 26.6 (s, 1C, C12), 25.0 (s, 1C, C15), 21.6 (s, 1C, C14), 20.1 (s, 1C, C16), 14.7 (s, 1C, C20) ppm. **HRMS-ESI⁺** (*m*/*z*): [M]²⁺ calcd for C₄₅H₄₅N₂PS²⁺, 338.1515; found, 338.1516.

### Synthesis of CH₃TAS

The synthesis of the CH₃TAS (**5**) is known to the person skilled in the art.

### Synthesis of CH₃TO dyes 4 and 6

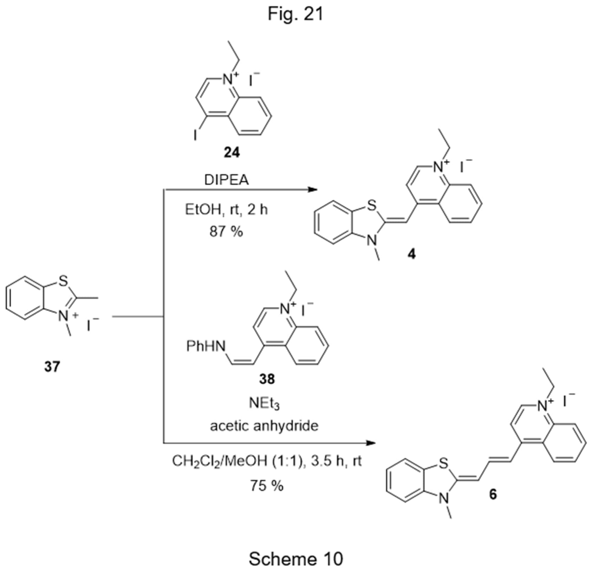

The Synthesis route of dyes CH₃TO-1 (**4**) and CH₃TO-3 (**6**), starting from benzothiazole **37,** is depicted in Fig. 21 (scheme 10).

### (Z)-1-ethyl-4-((3-methylbenzo[d]thiazol-2(3H)-ylidene)methyl)quinolin-1-ium (4):

Benzothiazolium **37** (117mg, 400 µmol, 1.00 eq) and quinilinium **24** (128 mg, 400 µmol, 1.00 eq) are suspended in ethanol (6.0 mL), diisopropylethylamine (0.15 mL, 884 µmol, 2.21 eq) is added and the solution stirred at room temperature in the dark for 2 h. The solvent is evaporated, and the crude purified by flash column chromatography (basic allox, MeOH in CH₂Cl₂: 0% → 0.2% → 0.5% → 0.7%) to yield the desired product 4 (156 mg, 350 µmol, 87 %) as red solid. ***R_{f}*** = 0.39 (CH₂Cl₂/MeOH 10:1 + 1 drop formic acid). **¹H-NMR** (500 MHz, DMSO-*d*₆, δ): 8.76 (d, 1H, ³*J* = 8.3 Hz, H-16), 8.64 (d, 1H, ³*J* = 7.1 Hz, H-21), 8.10 (d, 1H, ³*J* = 8.6 Hz, H-18), 8.01 (d, 1H, ³*J* = 7.8 Hz, H-3), 7.96 (t, 1H, ³*J* = 7.9 Hz, H-20), 7.75 - 7.70 (m, 2H, H-19, H-6), 7.56 (t, 1H, ³*J* = 7.5 Hz, H-1), 7.37 (t, 1H, ³*J* = 7.5 Hz, H-2), 7.29 (d, 1H, ³*J* = 7.1 Hz, H-17), 6.85 (s, 1H, H-10), 4.61 (q, 2H, ³*J* = 7.2 Hz, H-22), 3,97 (s, 3H, H-11), 1.46 (t, 3H, ³*J* = 7.2 Hz, H-23) ppm.**¹³C-NMR** (75 MHz, DMSO-*d*₆, δ): 159.8 (C-12), 148.4 (C-8), 143.93 (C-21), 140.4 (C-14), 136.8 (C-4), 133.3 (C-20), 128.1 (C-1), 126.8 (C-19), 125.9 (C-16), 124.4 (C-2), 124.2 (C-13), 123.8 (C-5), 122.9 (C-3), 118.0 (C-18), 112.9 (C-6), 108.0 (C-17), 88.0 (C-10), 49.4 (C-22), 33.9 (C-11), 14.7 (C-23) ppm. **HRMS-ESI⁺** (*m*/*z*): [M]⁺ calcd. for C₂₀H₁₉N₂S⁺: 319.1263, found: 319.1259.

### (Z)-1-ethyl-4-((3-methylbenzo[d]thiazol-2(3H)-ylidene)methyl)quinolin-1-ium (6):

Benzothiazolium **37** (147 mg, 505 µmol, 1.26 eq) and quinilinium **38** (161 mg, 400 µmol, 1.00 eq) are dissolved in CH₂Cl₂/Methanol (1:1, 4 mL), Acetic anhydride (0.4 mL) and triethylamine (0.4 mL) are added and the reaction is stirred for 3.5 h at room temperature. The crude dye is precipitated by pouring the reaction mixture in diethylether. The precipitate is washed with diethylether and purified by flash column chromatography (basic allox, MeOH in CH₂Cl₂: 0% → 0.2% → 0.5% → 0.7%) to yield the desired product **6** (178 mg, 377 µmol, 75 %) as dark blue solid. *R_{f} =* 0.31 (CH₂Cl₂/MeOH 10:1 + 1 drop formic acid). **¹H-NMR** (500 MHz, DMSO-*d*₆, δ): 8.44 (d, 1H, ³*J* = 8.6 Hz, H-23), 8.42 (d, 1H, ³*J* = 7.2 Hz, H-17), 8.11 (t, 1H, ³*J* = 12.8 Hz, H-11), 8.06 (d, 1H, ³*J* = 8.8 Hz, H-20), 7.94 (t, 1H, ³*J* = 7.5 Hz, H-22), 7.84 (t, 2H, ³*J* = 7.0 Hz, H-18, H-3), 7.69 (t, 1H, ³*J* = 7.7 Hz, H-21), 7.54 (d, 1H, ³*J* = 8.2 Hz, H-6), 7.44 (t, 1H, ³*J* = 7.3 Hz, H-2), 7.27 (t, 1H, ³*J* = 7.5 Hz, H-1), 7.08 (d, 1H, ³*J* = 13.3 Hz, H-12), 6.47 (d, 1H, , ³*J* = 12.3 Hz, H-10), 4.58 (q, 2H, ³*J* = 7.0 Hz, H-24), 3.71 (s, 3H, H-19), 1.44 (t, 3H, ³*J* = 7.0 Hz, H-25) ppm. **¹³C-NMR** (75 MHz, DMSO-*d*₆, δ): 161.5 (C-13), 150.3 (C-8), 143.7 (C-11), 142.2 (C-17), 141.9 (C-15), 137.7 (C-4), 133.4 (C-22), 127.6 (C-2), 126.7 (C-21), 125.2 (C-23), 124.2 (C-14), 124.1 (C-1), 122.5 (C-18), 117.8 (C-20), 112.4 (C-6), 109.3 (C-12), 98.8 (C-10), 49.3 (C-24), 32.9 (C-19), 14.7 (C-25) ppm. **HRMS-ESI⁺** *(m*/*z):* [M]⁺ calcd. for C₂₂H₂₁N₂S⁺: 345.1420, found: 345.1417.

### Synthesis of 6-TramTAS

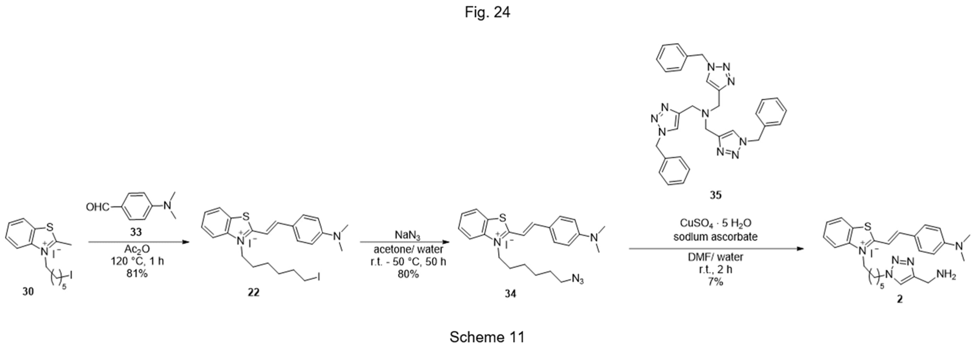

This synthesis is based on the scaffold of cyanine dyes described within the state of the art (cf. Fig. 24, scheme 11).

### (E)-2-(4-(dimethylamino)styryl)-3-(6-iodohexyl)benzo[d]thiazol-3-ium (6-IodoTAS, 22) :

Benzothiazolium (**30,** 1.00 g, 2.05 mmol) and dimethyl aminobenzaldehyd (306 mg, 2.05 mmol) are dissolved in Ac₂O (26.8 mL) and the mixture stirred at 120°C for 1 h. Water (26.8 mL) is added and the mixture stirred at 100 °C for 30 min. The reaction mixture was cooled to 25°C, the solvent removed, the product is filtered off and washed with acetone to yield the desired product (**22,** 1.03 mg, 1.67 mmol, 81%) as purple solid. **R*_{f}*** = 0.42 (CH₂Cl₂/MeOH 10:1). **¹H NMR** (300 MHz, DMSO-*d*₆, δ): 8.30 (d, 1H, *J* = 7.9 Hz, CH-4), 8.16 - 8.05 (m, 2H, CH-1, CH-11), 7.92 (d, 2H, *J =* 8.9 Hz, CH-14, CH-14'), 7.82 - 7.74 (m, 1H, CH-2), 7.72 - 7.64 (m, 1H, CH-3), 7.60 (d, 1H, *J =* 15.3 Hz, CH-12), 6.86 (d, 2H, *J* = 8.9 Hz, C*H*-13, C*H*-13'), 4.79 (t, 2H, *J =* 7.3 Hz, C*H*₂-5), 3.25 (t, 2H, *J* = 6.8 Hz, C*H*₂-10), 3.12 (s, 6H, C*H*₃-15, C*H*₃-15'), 1.88-1.78 (m, 2H, C*H*₂-6), 1.78-1.67 (m, 2H, C*H*₂-9), 1.53-1.35 (m, 4H, C*H*₂-7, C*H*₂-8). **¹³C NMR** (75 MHz, DMSO-d₆, δ): 171.0 (C_{q}), 153.5 (C_{q}), 150.5 (CH), 141.0 (C_{q}), 132.9 (2C, CH), 128.9 (CH), 127.3 (CH), 127.0 (C_{q}), 123.9 (CH), 121.4 (C_{q}), 115.8 (CH), 111.9 (2C, CH), 105.6 (CH), 47.8 (CH₂), 39.7 (2C, CH₃), 32.6 (CH₂), 29.4 (CH₂), 28.1 (CH₂), 24.6 (CH₂), 8.6 (CH₂). **HRMS-ESI⁺** (*m*/*z*): [M]⁺ calcd for C₂₃H₂₈IN₂S⁺, 491.1012; found, 491.1008.

### (E)-3-(6-azidohexyl)-2-(4-(dimethylamino)styryl)benzo[d]thiazol-3-ium (6-azindoTAS, 34) :

lodo-derivative (**22**, 500 mg, 0.81 mmol) and sodium azide (210 mg, 3.24 mmol) are dissolved in acetone (3.3 mL) and water (3.3 mL) and the mixture is stirred at r.t. for 17 h. Sodium azide (105 mg, 1.62 mmol) is added and the mixture is stirred at r.t. 25 h. Sodium azide (210 mg, 3.24 mmol) is added and the mixture is stirred at 50 °C for 3 h. The solvent is evaporated and the crude extracted in CH₂Cl₂ and water. The organic layers are dried over MgSO₄ and purified by flash column chromatography (allox, MeOH in CH₂Cl₂: 0.2% → 0.5%→ 1.0% → 5.0%→ 10%) to yield the desired product (**34**, 350 mg, 0.65 mmol, 80%) as purple solid. **R*_{f}*** = 0.38 (CH₂Cl₂/MeOH 10:1). **¹H NMR** (300 MHz, DMSO-*d*₆, δ): 8.31 (d, 1H, *J* = 7.9 Hz, C*H*-4), 8.17 - 8.05 (m, 2H, C*H-*1, C*H*-11), 7.93 (d, 2H, *J* = 8.8 Hz, C*H*-14, C*H*-14'), 7.82 - 7.74 (m, 1H, C*H*-2), 7.72 - 7.57 (m, 2H, C*H*-3, C*H*-12), 6.85 (d, 2H, *J* = 8.8 Hz, C*H*-13, C*H*-13'), 4.80 (t, 2H, *J* = 7.1 Hz, C*H*₂-5), 3.31 - 3.24 (m, 2H, C*H*₂-10), 3.12 (s, 6H, C*H*₃-15, C*H*₃-15'), 1.89-1.74 (m, 2H, C*H*₂-6), 1.53-1.36 (m, 6H, C*H*₂-7, C*H*₂-8, C*H*₂-9). **¹³C NMR** (75 MHz, DMSO-d₆, δ): 171.1 (C_{q}), 153.5 (C_{q}), 150.5 (CH), 141.1 (C_{q}), 132.9 (2C, CH), 128.9 (CH), 127.4 (CH), 127.0 (C_{q}), 123.9 (CH), 121.4 (C_{q}), 115.9 (CH), 111.9 (2C, CH), 105.7 (CH), 50.5 (CH₂), 47.8 (CH₂), 39.7 (2C, CH₃), 28.2 (CH₂), 28.0 (CH₂), 25.7 (CH₂), 25.2 (CH₂). **HRMS-ESI⁺** *(m*/*z):* [M]⁺ calcd for C₂₃H₂₈N₅S⁺, 406.2060; found, 406.2059.

**(E)-3-(6-(4-(aminomethyl)-1H-1,2,3-triazol-1-yl)hexyl)-2-(4-(dimethylamino)styryl)benzo[d]thiazol-3-ium (6-TramTAS, 2):** 6-azindoTAS (**34**, 41.9 mg, 78.5 µmol, 1.00 equiv.), copper sulfate pentahydrate (13.4 mg, 53.7 µmol, 0.68 equiv.), sodium ascorbate (10.6 mg, 53.5 µmol, 0.68 equiv.) and TBTA (**35**, 7.6 mg, 14.3 µmol, 0.18 equiv.) are dissolved in water (0.8 mL) and DMF (2.4 mL). Propargylamine (48 µL, 749 µmol, 9.54 equiv.) is added and the reaction mixture is stirred at room temperature in the dark for 2 h. Afterward, the reaction mixture is treated with aqueous sodium sulfide solution and filtered over Celite. After removal of the solvent under reduced pressure the crude is purified by preparative HPLC (Varian 5-35%) to yield the desired product (**2,** 3.3 mg, 5.74 µmol, 7%) as dark red solid. **R*ₜ*** = 19.7 min (gradient 1); **¹H-NMR** (500 MHz, DMSO-*d*₆) δ = 8.30 (dd, *³J_{H-H} =* 8.1 Hz, *⁴J_{H-H} =* 0.8 Hz, 1H, H3), 8.28 (s, 2H, H33), 8.10 (m, 3H, H1, H2, H3), 7.92 (d, *³J_{H-H} =* 9.0 Hz, 2H, H17, H21), 7.78 (m, 1H, H20), 7.68 (t, *³J_{H-H} =* 7.7 Hz, 1H, H18), 7.60 (d, *³J_{H-H} =* 15.2 Hz, H6), 6.85 (d, *³J_{H-H} =* 9.0 Hz, 2H, H22, H23), 4.79 (t, *³J_{H-H} =* 7.4 Hz, 2H, H10), 4.37 (t, *³J_{H-H} =* 7.1 Hz, 2H, H15), 4.10 (q, *³J_{H-H} =* 5.6 Hz, 2H, H32), 3.12 (s, 6H, H25, H26), 1.78 (dt, *³J_{H-H} =* 14.6 Hz, *³J_{H-H} =* 7.2 Hz, 4H, H11, H14), 1.46 (m, 2H, H12), 1.30 (m, 2H, H13) ppm; **¹³C-NMR** (125.76 MHz, DMSO-*d*₆) δ = 171.2 (s, 1C, C8), 153.6 (s, 1C, C19), 150.6 (s, 1C, C1), 141.2 (s, 1C, C4), 140.0 (s, 1C, C31), 133.0 (s, 1C, C2), 128.9 (s, 1C, C5), 127.5 (s, 1C, C18), 127.1 (s, 1C, C20), 124.1 (s, 2C, C17, C21), 124.0 (s, 1C, C16), 121.4 (s, 1C, C3), 115.9 (s, 1C, C30), 112.0 (s, 2C, C22, C23), 105.7 (s, 1C, C6), 49.3 (s, 1C, C15), 47.8 (s, 1C, C10), 40.0 (s, 2C, C25, C26), 33.9 (s, 1C, C32), 29.6 (s, 1C, C11), 28.2 (s, 1C, C14), 25.5 (s, 1C, C13), 25.2 (s, 1C, C12) ppm; **HRMS (ESI⁺)** m/z: calculated for C₂₆H₃₃N₆S: 461.2500; found: 461.2481.

### Synthesis of yellow PPh3 dye 16

Under exclusion of light 88.0 mg of **22** (0.14 mmol, 1.00 eq.) is dissolved in 1.8 mL MeCN. 112 mg of PPh₃ (0.43 mmol, 3.00 eq.) is added. The reaction mixture is heated to 85 °C for 3 h. After concentrating to dryness, the crude product is purified by preparative HPLC (gradient: 25-40% MeCN in water over 40 min). 90 mg of **16** (0.10 mmol, 72%) were obtained as dark red solid. **R*_{f}*** = 0.22 (DCM/MeOH 10:1); **¹H-NMR (500 MHz, DMSO-*d*₆) *δ*** = 8.31 (d, *³J_{H-H} =* 8.1 Hz, 1H, H3), 8.09 (t, *³J_{H-H} =* 11.6 Hz, 2H, H18, H22), 7.91 *(d, ³J_{H-H} =* 9.0 Hz, 2H, H19, H21), 7.87 (m, 3H, H33, H38, H43), 7.75 (m, 12H, H31, H32, H34 - 37, H39 - 42, H44, H45), 7.72 (d, *³J_{H-H}* = 3.9 Hz, 1H, H2), 7.68 (m, 1H, H1), 7.58 (d, *³J_{H-H} =* 15.2 Hz, 1H, H6), 6.81 (d, *³J_{H-H} =* 9.1 Hz, 2H, H23, H24), 4.76 (t, *³J_{H-H} =* 7.4 Hz, 2H, H10), 3.54 (t, *³J_{H-H} =* 11.3 Hz, 2H, H15), 3.11 (s, 6H, H26, H27), 1.74 (m, 2H, H11), 1.47 (m, 6H, H12 - 14) ppm; **¹³C-NMR (126 MHz, DMSO-*d*₆)** *δ* = 170.8 (s, 1C,C8), 153.2 (s, 1C, C20), 150.6 (s, 1C, C22), 141.0 (s, 1C, C4), 134.9 (d, 3C, C33, C38, C43), 133.6 (d, 6C, C31, C35, C36, C40, C41, C45), 133.0 (s, 2C, C19, C21), 130.3 (d, 6C, C32, C34, C37, C39, C42, C44), 129.0 (s, 1C, C2), 127.5 (s, 1C, C1), 129.0 (s, 1C, C5), 127.1 (s, 1C, C17), 124.0 (s, 1C, C3), 119.1 (s, 3C, C28-C30), 115.9 (s, 1C, C18), 111.9 (s, 2C, C23, C24), 105.7 (s, 1C, C6), 47.8 (s, 1C, C10), 39.8 (s, 2C, C26, C27), 29.4 (d, 1C, C12), 28.1 (s, 1C, C11), 24.8 (s, 1C, C13), 21.6 (d, 1C, C14), 20.1 (d, 1C, C15) ppm; **HRMS (ESI⁺)** m/z: calculated for C₄₁H₄₂N₂PS: 626.2900; found: 625.2806.

### Synthesis of yellow 6-AzTO-2 (40):

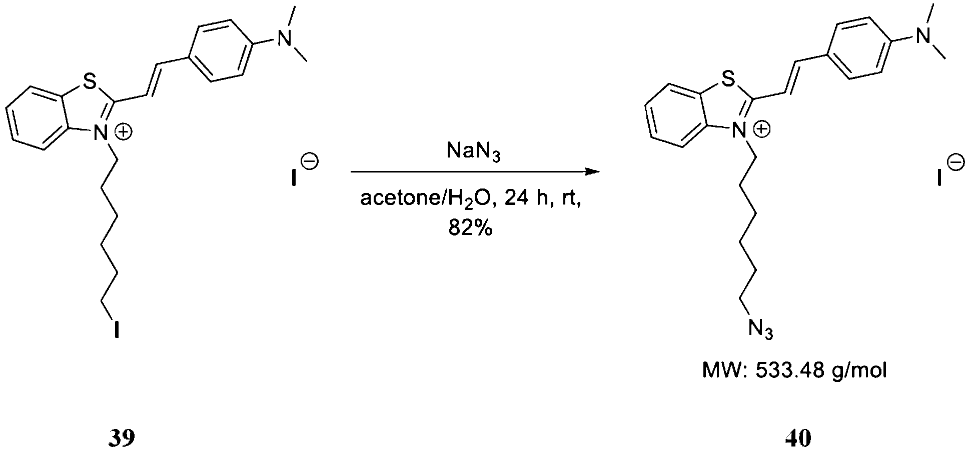

**Yellow 6-AzTO-2 (40):** Under exclusion of light and N₂-atmosphere 1.13 g of **39** (1.82 mmol. 1.00 eq.) and 1.18 g NaN₃ (18.2mmol, 10.0 eq.) was dissolved in in 14.0 mL acetone/water (1:1) and stirred for 24 h at room temperature. The reaction mixture was heated to 50 °C for 1 h and further 2.00 eq. NaN₃ (0.24 g, 3.64 mmol) was added. The dark red solution was stirred at 50 °C for 1.5 h, 16 h at room temperature and again at 50 °C for 3 h. The solvent was evaporated and the residue dissolved in DCM and an aqueous NaCO₃ solution (1:1, 20 mL). The aqueous layer was extracted with DCM (2x 10 mL). The combined organic layers was washed with 5 mL brine, dried over MgSO₄, and concentrated to dryness under reduced pressure. The crude product was purified by flash column chromatography (gradient of MeOH in DCM: 0% → 0.2% → 0,5% → 1% → 5% → 10%). 0.8 g of 40 (14.9 mmol, 82%) were obtained as dark red solid. **R_{F}** = 0.64 (DCM/MeOH 10: 1): **¹H-NMR (500 MHz, DMSO-*d*₆) *δ*** = 8.31 (d, *³J_{H-H} =* 8.0 Hz, 1H. H3). 8.14 (d, *³J_{H-H}=* 8.4 Hz, 1H, H22), 8.09 (d. *³J_{H-H}=* 15.2 Hz, 1H, H18), 7.93 (d, *³J_{H-H}=* 8.8 Hz, 2H. H19, H21), 7.78 *(t, ³J_{H-H}=* 7.7 Hz, 1H. H2). 7.68 (d, *³J_{H- H} =* 7.6 Hz, 1H, H1), 7.61 (d, *³J_{H-H} =* 15.2 Hz, 1H. H6), 6.85 (d, *³J_{H-H} =* 8.8 Hz, 2H. H23, H24), 4.80 (t, *³J_{H-H}* = 7.3 Hz, 2H, H10), 3.29 (t, *³J_{H-H} =* 6.9 Hz, 2H, H15), 3.12 (s, 6H, H26, H27), 1.81 (m. 4H, H11), 1.45 (m, 4H, H12 - 14) ppm: **¹³C-NMR** (500 **MHz, DMSO-*d*₆)** *δ* = 171.1 (s, 1C, C8), 153.6 (s, 1C, C20), 150.6 (s, 1C, C18), 141.1 (s, 1C, C4), 132.9 (s, 2C, C19, C21) 128.9 (s, 1C, C2), 127.4 (s, 1C, C1). 127.1 (s, 1C, C17), 123.9 (s, 1C, C3), 121.4 (s, 1C, C5), 115.9 (s, 1C, *C22),* 111.9 (s, 2C, C23, C24), 105.7 (s, 1C, *C6),* 50.5 (s, 2C, C26, C27), 47.8 (s, 1C, C10), 40.0 (s, 1C, C15), 28.2 (s, 1C, C11), 28.0(s. 1C, C14), 25.8 (s. 1C, C12), 25.2 (s, 1C, C13) ppm: **HRMS (ESI⁻)** m/z: calculated for C₂₃H₂₈N₅S: 406.2100: found: 406.2061.

### Synthesis of yellow 6-TramTO (42):

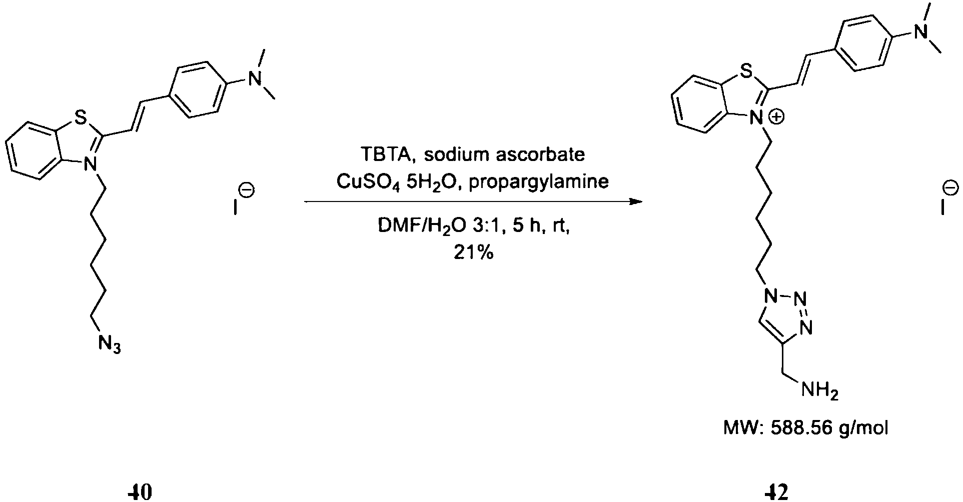

**Yellow 6-TramTO (42):** Under exclusion of light and stirring 6.9 mg CuSO₄ · 5 H₂O (28.0 µmol, 0.13 eq.) and 17.7 mg 54 (33.0 µmol, 0.15 eq.) were dissolved in 16.0 mL DMF/water (3:1). After 5 min 10 µL propargylamine (12.2 mg, 0.22 nunol, 1.00 eq.) was added. After further 5 min 119 mg of **40** (0.22 mmol, 1.00 eq.) was added to the green/blue solution. After further 5 min 11.0 mg sodium ascorbate (56.0 µmol, 0.25 eq.) was added. The dark red solution was stirred for 5 h at room temperature. The solvent was evaporated and the residue was dissolved in MeCN. The red solution was filtered over *Celite* and the solvent was evaporated. The crude product was purified by preparative HPLC (MeCN in H₂O (gradient: 15 - 35% over 40 min)). The different fractions were lyophilized to obtain 24.8 mg of product **42** (42.1 µmol, 21%) as a red solid. **R_{F}** = 0.60 (DCM/MeOH 10:1); **¹H-NMR** (500 MHz, DMSO-*d*₆) *δ* = 8.30 (dd, *³J_{H-H}=* 8.1 Hz, *⁴J_{H-H} =* 0.8 Hz. 1H, H3), 8.28 (s, 2H. H33). 8.10 (m, 3H, H1, H2, H3), 7.92 (d, *³J_{H-H}=* 9.0 Hz, 2H, H17, H21), 7.78 (m, 1H, H20). 7.68 (t, *³J_{H-H}=* 7.7 Hz, 1H. H18), 7.60 *(d. ³J_{H-H} =* 15.2 Hz. H6), 6.85 (d. *³J_{H-H}=* 9.0 Hz, 2H, H22, H23). 4.79 *(t, ³J_{H-H}* = 7.4 Hz. 2H, H10), 4.37 (t, *³J_{H-H} =* 7.1 Hz, 2H, H15), 4.10 *(q, ³J_{H-H} =* 5.6 Hz, 2H, H32), 3.12 (s, 6H, H25, H26), 1.78 (dt, *³J_{H-H} =* 14.6 Hz, *³J_{H-H} =* 7.2 Hz, 4H, H11, H14). 1.46 (m. 2H, H12), 1.30 (m, 2H, H13) ppm: **¹³C-NMR** (125.76 MHz, DMSO-d₆) *δ* = 171.2 (s, 1C, C8), 153.6 (s, 1C, C19), 150.6 (s, 1C, C1), 141.2 (s. 1C, C4), 140.0 (s, 1C, *C31).* 133.0 (s, 1C, C2), 128.9 (s, 1C, C5). 127.5 (s, 1C, C18), 127.1 (s, 1C, C20), 124.1 (s, 2C, C17, C21), 124.0 (s, 1C, C16), 121.4 (s, 1C, *C3),* 115.9 (s. 1C, C30), 112.0 (s, 2C, C22, C23), 105.7 (s, 1C, C6), 49.3 (s, 1C, C15), 47.8 (s, 1C, C10). 40.0 (s, 2C, *C25,* C26). 33.9 (s, 1C, C32), 29.6 (s, 1C, C11), 28.2 (s, 1C, C14), 25.5 (s, 1C, C13), 25.2 (s, 1C, C12) ppm: **HRMS (ESI⁺)** m/z: calculated for C₂₆H₃₃N₆S: 461.2500; found: 461.2481.

### List of abbreviations

Besides abbreviations, well known to any person of ordinary skill in the art, following table 3 lists some further abbreviations used herein.

**Table 3: Abbreviations used herein**

| | |
|---|---|
| Cha | L-cyclohexylalanine |
| dArg | D-arginine |
| MFI | mean fluorescence intensity |
| MOI | multiplicity of infection |
| MPP | mitochondria penetrating peptide |
| NT | nucleoli tracker |
| RFU | relative fluorescence untis |
| TBTA | tris((1-benzyl-4-triazolyl)methyl)amine |
| TO | thiazole orange |
| TAS | benzothiazole AminoStyryl |

### Description of the drawings

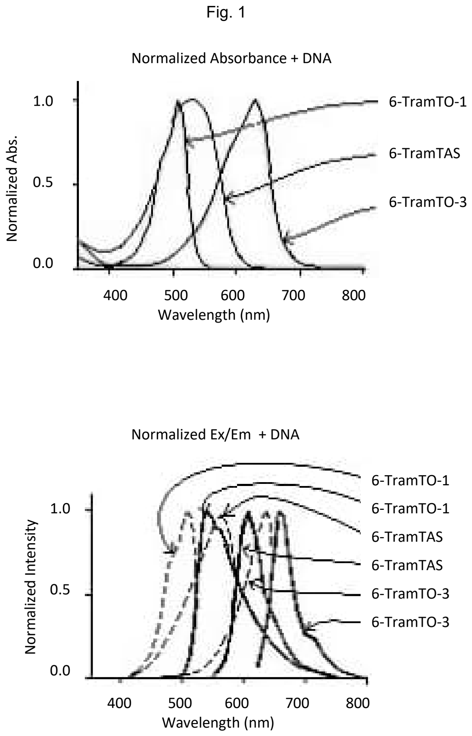

- Fig. 1:: Fig. 1 exemplarily shows Normalized spectral properties of TramTO dyes (1-3) in 10 mM Tris buffer, 50 mM KCI, pH= 7.6; DNA is dsDNA_{CT} double-stranded calf thymus DNA.
- Fig. 2:: Fig. 2 exemplarily shows bacterial growth curves at 2.5 µM and 5 µM dye concentration and with DMSO control (final DMSO concentration 0.4%). Mean of three measurements in triplicate.
- Fig. 3:: Fig. 3 exemplarily shows the viability of HeLa cells stained with cyanine dyes.
- Fig. 4:: Fig. 4 exemplarily shows the results of flow-cytometric measurements with *E*. *Coli* and *B. Subtilis.* Each experiment is labelled via subtitle, the subtitles indicating the following parameter settings (using equipment LSR Fortressa cell analyser): "Blue B-A" indicates an excitation wavelength of 488 nm, emission filter of 575/25 nm; "Blue Green A-A" indicates an excitation wavelength of 514 nm, emission filter of 610/20 nm; "Yellowgreen A-A" indicates an excitation wavelength of 561 nm, leading to an emission filter 685/35 nm;
- Fig. 5A:: The graphs of Fig. 5A show that for 6-TramTO-1 (1) there is clear labelling when added 5 µM of the dye for only 5 min before the measurements, dead cells (treated with 70% isopropanol for 1h, then washed with saline 3x) are labelled about 10x stronger: MFI 135 vs 1337. Additionally to the parameter settings regarding excitation/emission described in the legend to Fig. 4 the following parameter settings are used: "Blue A-A" indicates an excitation wavelength of 488 nm, emission filter of 670/30 nm; "Blue C-A" indicates an excitation wavelength of 488 nm, emission filter of 510/20 nm; "Blue Green B-A" indicates an excitation wavelength of 514 nm, emission filter of 542/27 nm; "Yellow Green B-A" indicates an excitation wavelength of 561 nm, emission filter of 632/22 nm.
- Fig. 5B:: The graphs of Fig. 5B show flow cytometric data of mixtures of dead and live E. Coli treated with left graph: PI (20 µM) and right graph: 6-TramTO-1 (1, 5 µM) for 5 min showing that the TramTO and PI give the same results. The parameter settings regarding excitation/emission (as far as they are applied) are equivalent to those described within the legend of Fig. 5A.
- Fig. 5C:: The graphs of Fig. 5C show dead bacteria treated with the two on the left 6-TramTO-1 (**1**) only and the two on the right PI only. (DMSO treated cells MFI: Blue C-A 4.26 and Yellow Green B-A 2.96). The value "262144" on the x-axis of each graph of Figs. 5B and 5C is always referring to the rightmost scale mark. The parameter settings regarding excitation/emission (as far as they are applied) are equivalent to those described within the legend of Fig. 5A.
- Fig. 6:: Fig. 6 exemplarily shows data regarding comparatively staining mixtures of dead and live cells with PI and 6-TramTO-1 (**1**). Upper density plot with green (Blue C-A) and orange (Yellow Green B-A) labelling in the axis and lower plots: all cells are labelled (right plot) compared to DMSO control (left plot). The parameter settings regarding excitation/emission (as far as they are applied) are equivalent to those described within the legend of Fig. 5A.
- Fig. 7:: Fig. 7 exemplarily shows experimental data proving that dual and even triple labelling is also possible with the TramTO dyes, using *Bacillus subtilis.* Suspensions of bacteria are labelled for 15 min with the different dyes at 5 µM, and are mixed in ratio 1:1 for dual labelling and in ratio 1:1:1 for the triple labelling experiments. (LSR Fortessa cell analyser). The value "262144" on the axes of each graph is always referring to the rightmost resp. uppermost scale mark. The parameter settings regarding excitation/emission (as far as they are applied) are equivalent to those described within the legend of Fig. 5A. The dyes applied are denoted on top of each graph, e.g. "**1 + 2**".
- Fig. 8:: Fig. 8 shows further examples of labelling, using *Klebsiella pneumoniae.* There the same protocol is applied (15 min labelling at 5 µM dye concentration). BD FACS Aria Ill. 100,000 events per sample are analysed. Left signal in each graph: DMSO/background fluorescence control. Channels: Alexa Fluor 488 (excitation wavelength of 488 nm, emission filter of 530/30 nm), PE-Texas Red (excitation wavelength of 561 nm, emission filter of 610/20 nm) and APC (excitation wavelength of 633 nm, emission filter of 660/20 nm).
- Fig. 9A:: Fig. 9A shows the results of phagocytosis studies with different bacterial strains labelled with 1 or 3. Here the following channels were used (apparatus "Guava easyCyte"): Green: excitation wavelength of 488 nm, emission filter of 525/30 nm; Yellow: excitation wavelength of 488 nm, emission filter of 582/26 nm; Red: excitation wavelength of 488 nm, emission filter of 611/319 nm; Red2: excitation wavelength of 640 nm, emission filter of 680/30 nm.
- Fig. 9B:: Fig. 9B shows examples of fluorescence microscopy pictures of stained HeLa cells; left: "green" 6-TramTO-1 (**1**), center: "yellow" 6-TramTAS (**2**); right: "red" 6-TramTO-3 (**3**), each one showing stained nuclei of fixed HeLa cells. (3 µM, 20 min)
- Fig. 9C:: Fig. 9C shows further examples of fluorescence microscopy pictures of stained HeLa cells (dyes in complete live cells: PPh₃-TOs (**15-17**) in living HeLa cells (staining being done with dye concentration = 3 µM (in FluoroBrite media), duration of staining being 20 min, the cells being rinsed afterwards and the images being acquired after further 30 min)). left: "green" cyanine dye **15,** center: "yellow" cyanine dye **16;** right: "red" cyanine dye **17.**
- Fig. 9D:: Fig. 9D shows exemplarily fluorescence microscopy pictures of bacterial labelling (5µM, 15 min for TramTO dyes, 1 µM for DAPI) using exemplarily *E*. *Coli* and *B*. *Subtilis.*
- Fig. 10:: Scheme 5 shows the general retrosynthetic approach for the preparation of different TO-derivatives (e.g. 6-xxxTO-1 and 6-xxxTO-3 derivatives, "xxx" denoting "Tram" or "lodo" or "Tri" or "PPh₃").
- Fig. 11:: Scheme 8 shows the synthesis route of 6-TramTO-1 (**1**) starting from benzothiazolium (**30**).
- Fig. 15:: Scheme 9 shows the synthesis routes of 6-PipTO-1 (**14**), 6-PipTO-3 (**10**), 6-TriTO-1 (**13**), 6-PPh3TO-1 (**15**) and 6-PPh3TO-3 (**17**) starting from 6-lodoTO-1 (**21**) or 6-lodoTO-3 (**23**).
- Fig. 21:: Scheme 10 shows the synthesis route of dyes CH₃TO-1 (**4**) and CH₃TO-3 (**6**), starting from benzothiazole **37**.
- Fig. 24:: Scheme 11 shows the Synthesis route of "yellow" 6-TramTAS (**2**) starting from benzothiazole (**30**).

## Claims

1. A method for staining living cells **characterized in that** the method comprises the following steps:
i) at least one cyanine dye is applied to at least one sample of cells by adding a solution of the at least one cyanine dye to the at least one sample of cells, thus providing at least one sample of cells,
being stained with at least one cyanine dye;
ii) incubating the at least one sample of cells according to step i) for at least 15 minutes, so that stained living cells are existent;
whereat the at least one cyanine dye according to step i) is a canine dye having the chemical structure according to general formula (I), 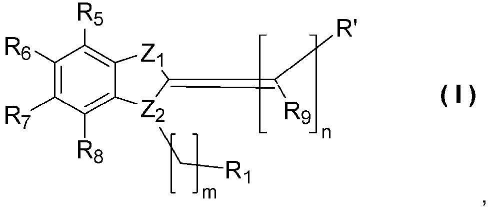 wherein
- R' is a moiety according to formula (II), 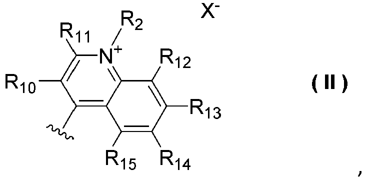
- R₂ is selected from the list comprising H, methyl, ethyl, propyl, iso-propyl, butyl, iso-butyl, tert-butyl, alkyl, alkinyl, alkylidene,
- R₅ to R₁₅ are independently from each other selected from the list comprising the substituents H, halogen, methyl. ethyl, propyl, isopropyl, methoxy, ethoxy, propoxy, amino, methylamino, ethylamino, monoalkylamino, dialkylamino, a nitro group, a sulfo group,
- Z₁ is S and Z₂ is N,
- X⁻ is selected from the list comprising halogenate, sulfate, triflate, trifluoro acetate, difluoro acetate or fluoro acetate,
- n is 1 to 3,
- m is 6,
**characterized by**
- R₁ is 1,2,3-triazol-4-(aminomethyl)-1-yl.

2. A method for staining living cells according to claim 1, **characterized in that** the method comprises the following steps i) and ii) and the additional step iii):
i) at least two cyanine dyes are applied to at least two separate samples of cells whereat each sample is stained with one of the at least two cyanine dyes by adding a solution of the cyanine dye to the sample of cells,
thus providing at least two separate samples of cells, each one being stained with at least one cyanine dye;
ii) each sample of cells according to modified step i) is incubated for at least 15 minutes, so that stained living cells are existent;
iii) the at least two separate samples of cells provided according to step ii),
each one being stained with at least one cyanine dye, are combined within one sample vessel with each other thus forming one combined sample.

3. A method according to claim 1 or claim 2, **characterized in that** the cyanine dye is a nucleic acid binding cyanine dye.

4. A method according to claim 1 or claim 2 or claim 3, **characterized in that** the method comprises the following additional preparation step before step i):
a) obtaining and isolation of at least one sample of cells to be introduced into step i), whereat this obtaining and isolation-step may include one or more steps for concentrating and/or breeding of the cells.

5. A kit for carrying out the method according to claim 1 or claim 2 or claim 3 or claim 4, the kit comprising the cyanine dye and optionally supportive substances.
